# EUROPEAN PATENT APPLICATION

(11) **EP 4 454 657 A1**
(43) Date of publication of application: **30.10.2024**
(21) Application number: 22912003.5
(22) Date of filing: 22.12.2022
(51) Int. Cl.: A61K 38/16, A61K 47/68, A61K 47/60, A61P 1/16, A61P 3/10

(54) **LIVER-TARGETED SUBSTANCE AND USE THEREOF**

(30) Priority: 22.12.2021 KR 20210185351
(71) Applicant: Hanmi Pharm. Co., Ltd., Hwaseong-si, Gyeonggi-do 18536 (KR)
(72) Inventor: KIM, Jung Kuk, Hwaseong-si Gyeonggi-do 18469 (KR); LEE, Jong Suk, Hwaseong-si Gyeonggi-do 18469 (KR); CHOI, Jae Hyuk, Hwaseong-si Gyeonggi-do 18469 (KR); LEE, Jong Min, Hwaseong-si Gyeonggi-do 18469 (KR); KIM, Sang Yun, Hwaseong-si Gyeonggi-do 18469 (KR)
(74) Representative: Regimbeau
(86) International application number: PCT/KR2022/021129
(87) International publication number: WO 2023/121370

(57) **Abstract**

The present invention relates to a liver-targeted drug, and therapeutic use thereof for diseases requiring drug action in the liver. In addition, the present invention relates to a method in which a substance having activity with respect to glucagon is used so as to induce targeting of liver tissues or an increase in distribution in liver tissues after *in vivo* administration.

## Description

### [Technical Field]

The present invention relates to a liver-targeted substance and therapeutic uses thereof for a disease requiring drug action in the liver. The present invention also relates to a method for inducing targeting of a liver-targeted substance to liver tissue or inducing an increased distribution of a liver-targeted substance in liver tissue after the *in vivo* administration.

### [Background Art]

The liver is one of the largest organs in our body and plays a key role in various and overall metabolism. Among the vegetable or animal materials that we consume and the metabolites resulting from biological function performance, some are beneficial to the body while many are harmful. The liver performs chemical processes that help the appropriate biological utilization of the beneficial materials, while helping in the safe excretion of the harmful materials through urine or feces by chemical metabolic processes. The liver plays an essential role in biological functions by synthesizing and secreting various types of proteins, fats, and carbohydrates required for our body. Therefore, the deterioration of liver function may cause several functional abnormalities. An insufficient production of coagulation factors involved in blood coagulation causes easy bleeding, resulting in frequent bleeding from weak gums and the like, and in cirrhosis patients, often causes hypoglycemia resulting from fasting due to poor insulin decomposition and insufficient glycogen storage in the liver. The liver also plays a central role in defense against bacterial invasion. Especially, Kupffer cells, among the cells in the liver, mainly consume foreign materials or bacteria by phagocytosis and induce natural immune actions in the body by exposing viruses entering the body to the immune system. Additionally, bile, a main substance synthesized and secreted in liver cells, is formed in approximately 800-1000 cc per day and is composed of water, electrolyte, bile acids, cholesterol, phospholipids, and bilirubin. As for the main functions of bile, bile acids in the bile play an important role in digesting and absorbing fat and fat-soluble vitamins in the small intestine, and bile itself serves to excrete many waste products produced in the body through feces. There are various diseases, such as liver disease, hypoglycemia, or congenital hyperinsulinism, which arise from a variety of causes and require drug action in the liver.

Meanwhile, in order to treat the above-mentioned liver diseases, allowing drugs to reach the liver and be distributed therein at high concentrations is one of the important technical challenges (Korean Patent Publication No. KR 10-2013-0131227).

A liver-targeted drug can optimize drug treatment by minimizing side effects of existing medicines and maximizing efficacy and effectiveness thereof, enabling efficient delivery of a required amount of drug. This increases the amount of drug that reaches a target site, improving bioavailability, thereby enabling more effective treatment. This also prevents the drug from being delivered to sites other than the intended one, thereby reducing side effects, and improves the patient's response to the drug, greatly contributing to the improvement in patient compliance.

### [Disclosure]

### [Technical Problem]

To enhance the therapeutic effect for a disease requiring drug action in the liver, there is a need for the development of a liver-targeted drug having an excellent distribution in the liver among the organs in the body of a subject to whom it is administered, as well as a therapeutic effect.

### [Technical Solution]

An aspect of the present invention is to provide a pharmaceutical composition containing a liver-targeted drug, specifically, a pharmaceutical composition containing a liver-targeted drug and having a high distribution of the drug in the liver among the organs in the body of a subject to whom it is administered.

Another aspect of the present invention is to provide a pharmaceutical composition containing a liver-targeted drug, specifically, a pharmaceutical composition containing a liver-targeted drug for preventing or treating a disease requiring drug action in the liver.

Another aspect of the present invention is to provide a method for preventing or treating a disease requiring drug action in the liver, the method including administering to a subject in need thereof the pharmaceutical composition or a biologically active substance targeted to liver tissue.

Another aspect of the present invention is to provide use of the liver-targeted drug, specifically, a biologically active substance targeted to liver tissue, in the preparation of a medication for prevention or treatment of a disease requiring drug action in the liver.

Another aspect of the present invention is to provide a method for inducing targeting to the liver by administering to a subject in need thereof the liver-targeted drug, specifically, a biologically active substance targeted to liver tissue.

Another aspect of the present invention is to provide a method for inducing an increased distribution of a biologically active substance in liver tissue by administering to a subject in need thereof the liver-targeted drug, specifically, a biologically active substance targeted to liver tissue.

### [Advantageous Effects]

The drug (e.g., a biologically active protein or a peptide derivative) of the present invention containing a substance binding to a receptor of liver cells is delivered from blood vessels to liver tissue more effectively to be provided as a biologically active substance that is relatively abundantly distributed in the liver among the organs in the body after *in vivo* administration, specifically, a biologically active substance that is relatively abundantly distributed in liver tissue by having binding affinity for the glucagon receptor, and furthermore shows a high distribution in the liver even within 7 days and thus can be applied to the treatment of a disease or the like requiring drug action in the liver, including a liver disease, hypoglycemia, and congenital hyperinsulinism, while reducing the patient's discomfort in drug administration.

### [Detailed Description of the Invention]

In accordance with an aspect of the present invention, there is provided a pharmaceutical composition containing a liver-targeted drug, specifically, a pharmaceutical composition having a high distribution of the drug in the liver among the organs in the body of a subject to whom it is administered. In an embodiment, the liver-targeted drug may be in the form of a peptide including an amino acid sequence of General Formula 1 below or a long-acting conjugate including the same. In another embodiment, the liver-targeted drug may be in the form of a peptide including an amino acid sequence of General Formula 2 below or a long-acting conjugate including the same.

In an embodiment, the liver-targeted drug may be in the form of a peptide including an amino acid sequence of General Formula 1 below:

X1-X2-QGTF-X7-SD-X10-S-X12-X13-X14-X15-X16-X17-X18-X19-X20-X21-F-X23-X24-W-L-X27-X28-X29-X30 (General Formula 1, SEQ ID NO: 46)

wherein,
X1 is histidine (H), desamino-histidyl, N-dimethyl-histidyl, beta-hydroxy imidazopropionyl, 4-imidazoacetyl, beta-carboxy imidazopropionyl, tryptophan (W), or tyrosine(Y), or is absent;
X2 is alpha-methyl-glutamic acid, aminoisobutyric acid (Aib), D-alanine, glycine (G), N-methylglycine (Sar), serine (S), or D-serine;
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is lysine (K) or cysteine (C);
X15 is aspartic acid (D), glutamic acid (E), or cysteine (C);
X16 is glutamic acid (E), aspartic acid (D), serine (S), alpha-methyl-glutamic acid, or cysteine (C), or is absent;
X17 is aspartic acid (D), glutamine (Q), glutamic acid (E), lysine (K), arginine (R), serine (S), cysteine (C), or valine (V), or is absent;
X18 is alanine (A), aspartic acid (D), glutamine (Q), glutamic acid (E), arginine (R), valine (V), or cysteine (C), or is absent;
X19 is alanine (A), arginine (R), serine (S), valine (V), or cysteine (C), or is absent;
X20 is lysine (K), histidine (H), glutamic acid (E), glutamine (Q), aspartic acid (D), arginine (R), alpha-methyl-glutamic acid, or cysteine (C), or is absent;
X21 is aspartic acid (D), glutamic acid (E), leucine (L), valine (V), or cysteine (C), or is absent;
X23 is isoleucine (I), valine (V), arginine (R), or is absent;
X24 is valine (V), arginine (R), alanine (A), cysteine (C), glutamate (E), lysine (K), glutamine (Q), alpha-methylglutamate, or leucine (L), or is present;
X27 is isoleucine (I), valine (V), alanine (A), lysine (K), methionine (M), glutamine (Q), or arginine (R), or is absent;
X28 is glutamine (Q), lysine (K), asparagine (N), or arginine (R), or is absent;
X29 is lysine (K), alanine (A), glycine (G), or threonine (T), or is absent; and
X30 is cysteine (C) or is absent.

In accordance with an aspect of the present invention, there is provided a pharmaceutical composition containing a liver-targeted drug and having a high distribution of the drug in the liver among the organs in the body of a subject to whom it is administered. In an embodiment, the liver-targeted drug may be in the form of a peptide including an amino acid sequence of General Formula 2 below:

Y-Aib-QGTF-X7-SD-X10-S-X12-Y-L-X15-X16-X17-R-A-X20-X21-F-V-X24-W-L-M-N-T-X30 (General Formula 2, SEQ ID NO: 47)

wherein in the General Formula 2 above,
X7 is threonine (T), valine (V), or cysteine (C);
X10 is tyrosine (Y) or cysteine (C);
X12 is lysine (K) or cysteine (C);
X15 is aspartic acid (D) or cysteine (C);
X16 is glutamic acid (E) or serine (S);
X17 is lysine (K) or arginine (R);
X20 is glutamine (Q) or lysine (K);
X21 is aspartic acid (D) or glutamic acid (E);
X24 is valine (V) or glutamine (Q); and
X30 is cysteine (C) or is absent
(with the proviso that an amino acid sequence of General Formula 2 identical to SEQ ID NO: 12 is excluded).

In another embodiment, the peptide may be in the form of a long-acting conjugate, wherein the long-acting conjugate is represented by Chemical Formula 1 below:

[Chemical Formula 1] X-L-F

wherein,
X represents a peptide including an amino acid sequence of General Formula 2 below:
L represents a linker containing an ethylene glycol repeating unit;
F represents an immunoglobulin Fc region; and
the "-" symbols represent covalent linkages between X and L and between L and F, respectively.

In the pharmaceutical composition according to any one of the previous embodiments, the organs in the body are liver, heart, lung, large intestine, spleen, pancreas, adipose tissue, small intestine, stomach, muscle, kidney, and brain, among which the distribution of the drug in the liver is highest.

In the pharmaceutical composition according to any one of the previous embodiments, the composition may be used for preventing or treating a disease requiring drug action in the liver.

In the pharmaceutical composition according to any one of the previous embodiments, the tissue-to-serum ratio (T/S ratio) of the liver-targeted drug in the liver after administration may be at least one selected from below: (a) a T/S ratio of 20-40% at 40-50 hours after administration; and (b) a T/S ratio of 25-40% at 160-180 hours after administration.

In the pharmaceutical composition according to any one of the previous embodiments, the T/S ratio of the liver-targeted drug in the liver after administration may be at least one selected from below: (a) a T/S ratio of 25-35% at 2 days after administration; and (c) a T/S ratio of 27-35% at 7 days after administration.

In the pharmaceutical composition according to any one of the previous embodiments, the liver-targeted drug may show a distribution ratio of 1: about 2 to about 4 in the liver to lung tissue after administration.

In the pharmaceutical composition according to any one of the previous embodiments, the liver-targeted drug may show a distribution ratio of 1: about 2.2 to about 3.2 in the liver to lung tissue after administration.

In the pharmaceutical composition according to any one of the previous embodiments, the distribution ratio in the liver to lung tissue after administration may be a distribution ratio at about 40 hours to about 180 hours after administration.

In the pharmaceutical composition according to any one of the previous embodiments, the distribution ratio in the liver to lung tissue after administration may be a distribution ratio at about 2 days to about 7 days after administration.

In the pharmaceutical composition according to any one of the previous embodiments, the liver-targeted drug shows (a) a distribution ratio of 1:1.6-3.0 in the liver to the heart at 40-50 hours after administration; and (b) a distribution ratio of 1 :2.8-7 in the liver to the heart at 160-180 hours after administration.

In the pharmaceutical composition according to any one of the previous embodiments, the liver-targeted drug may be in the form of a peptide or a long-acting conjugate including the same, which is relatively abundantly distributed in the liver among the liver among the organs in the body after administration.

In the pharmaceutical composition according to any one of the previous embodiments, the liver-targeted drug may have therapeutic activity on a disease requiring drug action in the liver.

In the pharmaceutical composition according to any one of the previous embodiments, the disease requiring drug action in the liver may be a liver disease, hypoglycemia, or congenital hyperinsulinism.

In the pharmaceutical composition according to any one of the previous embodiments, the hypoglycemia may be acute hypoglycemia or chronic hypoglycemia.

In the pharmaceutical composition according to any one of the previous embodiments, the liver-targeted drug may be a protein or peptide including a sequence of a peptide having binding affinity for the glucagon receptor.

In the pharmaceutical composition according to any one of the previous embodiments, a ring may be formed between the amino acids of X16 and X20 in General Formula 2.

In the pharmaceutical composition according to any one of the previous embodiments, the peptide including an amino acid sequence of General Formula 2 may include an amino acid sequence selected from the group consisting of SEQ ID NOS: 13, 15, 19, 33, and 36 to 45.

In the pharmaceutical composition according to any one of the previous embodiments, the peptide including an amino acid sequence of General Formula 2 may include an amino acid sequence selected from the group consisting of SEQ ID NOS: 33 and 36 to 44.

In the pharmaceutical composition according to any one of the previous embodiments, the peptide including an amino acid sequence of General Formula 1 may include an amino acid sequence of any one of SEQ ID NOS: 20, 22, 23, 27, 33, 35, 37, 38, 40, 41, 42, and 44.

In the pharmaceutical composition according to any one of the previous embodiments, the peptide including an amino acid sequence of General Formula 1 may include an amino acid sequence of any one of SEQ ID NOS: 20, 22, 23, 27, 33, 37, 38, and 44.

In the composition according to any one of the previous embodiments, each amino acid of at least one amino acid pair among the amino acid pairs of X10 and X14, X12 and X16, X16 and X20, X17 and X21, X20 and X24, and X24 and X28 in General Formula 1 may be substituted with glutamic acid or lysine capable of forming a ring.

In the composition according to any one of the previous embodiments, each amino acid of the amino acid pair of X12 and X16, the amino acid pair of X16 and X20, or the amino acid pair of X17 and X21 may be substituted with glutamic acid or lysine capable of forming a ring.

In the composition according to any one of the previous embodiments, a ring may be formed between both amino acids of at least one amino acid pair among the amino acid pairs of X10 and X14, X12 and X16, X16 and X20, X17 and X21, X20 and X24, and X24 and X28 in General Formula 1.

In the composition according to any one of the previous embodiments, the peptide may include an amino acid sequence selected from the group consisting of SEQ ID NOS: 1 to 44.

In the composition according to any one of the previous embodiments, the C-terminus of the peptide may be amidated or have a free carboxy group (-COOH).

In the composition according to any one of the previous embodiments, the C-terminus of the peptide may be amidated.

In the composition according to any one of the previous embodiments, the C-terminus of the peptide may not be modified.

In the composition according to any one of the previous embodiments, the chemical formula weight of an ethylene glycol repeating unit region in L may be in the range of 1 to 100 kDa.

In the composition according to any one of the previous embodiments, the structure of Chemical Formula 1 may be a structure of Chemical Formula 3 below: 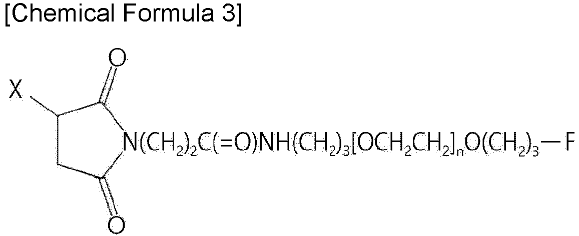 wherein, X and F are as defined in Chemical Formula 1.

In the composition according to any one of the previous embodiments, the ethylene glycol repeating units may be [OCH₂CH₂]n where n is a natural number, wherein n is determined such that the average molecular weight, for example, the number average molecular weight, of the [OCH₂CH₂]n region in the peptide conjugate is 1 to 100 kDa.

In the composition according to any one of the previous embodiments, the value of n may be determined such that the average molecular weight, for example, the number average molecular weight, of the [OCH₂CH₂]n region in the peptide conjugate is 10 kDa.

In the pharmaceutical composition according to any one of the previous embodiments, L may be polyethylene glycol.

In the composition according to any one of the previous embodiments, X may be linked through a sulfur atom of cysteine in the peptide.

In the pharmaceutical composition according to any one of the previous embodiments, F may be an IgG Fc region.

In the pharmaceutical composition according to any one of the previous embodiments, the immunoglobulin Fc region may be derived from IgG4.

In the composition according to any one of the previous embodiments, F may have a structure in which two polypeptide chains are linked via a disulfide bond, wherein the linking may be made through only a nitrogen atom of one chain of the two chains.

In the composition according to any one of the previous embodiments, F may include a monomer, which is the amino acid sequence of SEQ ID NO: 70.

In the composition according to any one of the previous embodiments, F may be a homodimer of monomers, each of which is the amino acid sequence of SEQ ID NO: 70.

In the composition according to any one of the previous embodiments, F may be a homodimer including the amino acid sequence of SEQ ID NO: 71.

In the composition according to any one of the previous embodiments, F may be linked through a nitrogen atom of N-terminus proline thereof.

In the composition according to any one of the previous embodiments, F, which is an immunoglobulin Fc region, and X may be aglycosylated.

In accordance with another aspect of the present invention, there is provided a biologically active substance targeted to liver tissue, specifically, a biologically active substance targeted to liver tissue and containing a substance having binding affinity for a receptor present in the liver.

In accordance with another aspect of the present invention, there is provided a method for preventing or treating a disease requiring drug action in the liver, the method including administering to a subject in need thereof the pharmaceutical composition or a biologically active substance targeted to liver tissue.

In accordance with another aspect of the present invention, there is provided use of the liver-targeted drug, specifically, a biologically active substance targeted to liver tissue, in the preparation of a medication for prevention or treatment of a disease requiring drug action in the liver.

In accordance with another aspect of the present invention, there is provided a method for inducing targeting to the liver by administering to a subject in need thereof the liver-targeted drug, specifically, a biologically active substance targeted to liver tissue.

In accordance with another aspect of the present invention, there is provided a method for inducing an increased distribution of the biologically active substance in liver tissue by administering to a subject in need thereof the liver-targeted drug, specifically, a biologically active substance targeted to liver tissue.

### [Detailed Description of the Invention]

Hereinafter, detailed contents for implementing the present invention will be described below. Each description and embodiment disclosed in the present application may also be applied to other descriptions and embodiments. That is, all combinations of various elements disclosed in the present application fall within the scope of the present disclosure. Furthermore, the scope of the present invention is not limited by the specific description below.

Furthermore, a skilled person in the art will recognize, or be able to ascertain, by using no more than routine experimentation, many equivalents to the specific embodiments of the invention described herein. Furthermore, these equivalents are intended to be included in the present invention. The contents of all the documents in the present specification are incorporated herein by reference. Furthermore, throughout the overall specification, many papers and patent documents are referenced and their citations are provided. The disclosures of cited papers and patent documents are entirely incorporated by reference herein, and the level of the technical field within which the present invention falls and details of the present invention are explained more clearly.

Throughout the description herein, not only the conventional one-letter and three-letter codes for naturally occurring amino acids, but also those three-letter codes generally allowed for other amino acids, such as α-aminoisobutyric acid (Aib), N-methylglycine (Sar), and α-methyl-glutamic acid, are used. The amino acids mentioned in abbreviations herein are described according to the IUPAC-IUB rules:

| | |
|---|---|
| alanine (Ala, A), | arginine (Arg, R) |
| asparagine (Asn, N), | aspartic acid (Asp, D) |
| cysteine (Cys, C), | glutamic acid (Glu, E) |
| glutamine (Gln, Q), | glycine (Gly, G) |
| histidine (His, H), | isoleucine (Ile, I) |
| leucine (Leu, L), | lysine (Lys, K) |
| methionine (Met, M), | phenylalanine (Phe, F) |
| proline (Pro, P), | serine (Ser, S) |
| threonine (Thr, T), | tryptophan (Trp, W) |
| tyrosine (Tyr, Y), | valine (Val, V) |

Herein, "Aib" may be used interchangeably with "2-aminoisobutyric acid" or "aminoisobutyric acid", and 2-aminoisobutyric acid and aminoisobutyric acid may be used interchangeably with each other.

An aspect of the present invention provides a composition, for example, a pharmaceutical composition, containing a liver-targeted drug. Specifically, an aspect of the present invention is directed to a pharmaceutical composition containing a liver-targeted drug and having a high distribution of the drug in the liver among the organs in the body of a subject to whom it is administered.

The high distribution of the drug in the liver among the organs in the body may mean that the distribution of the liver-targeted drug after administration of the liver-targeted drug into the body is highest in the liver among the organs in the body including the liver, heart, lung, large intestine, spleen, pancreas, adipose tissue, small intestine, stomach, muscle, kidney, and brain, but is not limited thereto.

The pharmaceutical composition may be used for the prevention or treatment of a disease requiring drug action in the liver.

The term "liver-targeted drug" as used herein refers to a drug that can be targeted to liver tissue. The meaning of being targeted to liver tissue may be that the distribution of the drug after administration is high in the liver compared with other organs, but is not limited thereto. The liver-targeted drug may retain therapeutic activity on a disease requiring drug action in the liver.

The tissue-to-serum ratio (T/S ratio) of the liver-targeted drug in the liver after administration may be at least one selected from below, but is not limited to: (a) a T/S ratio of about 20% to about 40%, about 24% to about 40%, about 25% to about 40%, about 25% to about 38%, about 25% to about 35%, about 25% to about 34%, about 25% to about 33%, about 26% to about 33%, about 26% to about 32%, about 27% to about 32%, about 28% to about 32%, about 29% to about 31%, or 29.5% to about 30.5% at about 40 hours to about 50 hours after administration; and (b) a T/S ratio of about 25% to about 40%, about 26% to about 38%, about 27% to about 35%, about 27% to about 34%, about 27% to about 33%, about 28% to about 33%, about 29% to about 33%, about 29% to about 32%, about 30% to about 32%, or about 30.5% to about 31.5% at about 160 hours to about 180 hours after administration. The above feature may be at least one or both of two selected from (a) or (b), but is not limited thereto.

The T/S ratio of the liver-targeted drug in the liver after administration may have at least one selected from below, but is not limited to: (a) a T/S ratio of about 25% to about 35%, about 26% to about 34%, about 27% to about 33%, about 28% to about 32%, about 29% to about 31%, about 29.5% to about 30.5%, and about 29.8% at about 2 days after administration; and (c) a T/S ratio of about 27% to about 35%, about 27% to about 34%, about 28% to about 33%, about 29% to about 32%, about 30% to about 31.5%, and about 31% at about 7 days after administration. The above feature may be at least one or both of two selected from (a) or (b), but is not limited thereto.

The tissue-to-serum ratio (T/S ratio), which is the ratio of concentrations in tissue to serum, is converted into a percentage and may be measured using a known method. For example, the tissue-to-serum ratio (T/S ratio) is calculated as tissue concentration/serum concentration X 100. Regarding the concentration measurement, the concentration of a substance is measured using an ELISA method or the like after organ extraction.

A higher T/S ratio means that a substance administered to the tissue shows a higher distribution compared with that in a tissue with a lower T/S ratio. In general, a drug administered into the body shows a high distribution thereof in the lungs and heart. Therefore, when the distribution in liver tissue is determined to be higher than those in the lung tissue and heart tissue by comparison of the distributions in the lung tissue and heart tissue with that in the liver tissue, this may mean that the liver-targeted drug of the present invention is effectively targeted to liver tissue. In addition to the lung and heart tissues, any tissue in which the T/S ratio can be compared with that in the liver tissue by measuring the T/S ratio capable of confirming that the liver-targeted drug is effectively targeted to liver tissue may be included without limitation.

The distribution ratio of the liver-targeted drug in the liver to lung tissue after administration may be 1 : about 2 to about 4.0, 1 : about 2 to about 3.5, 1 : about 2.1 to about 3.4, 1 : about 2.2 to 3.2, 1 : about 2.2 to about 3.3, 1 : about 2.3 to about 3.2, 1 : about 2.4 to about 3.2, 1 : about 2.4 to about 3.1, 1 : about 2.4 to about 3.0, 1 : about 2.5 to about 2.95, 1 : about 2.5 to 2.9, 1 : about 2.55 to about 2.87, 1 : about 2.6 to 2.9, or 1: about 2.5 to 3.9, but is not limited thereto.

The distribution ratio may be measured on the basis of T/S (%), and when the T/S (%) in the lung is set to 1 as a reference value, the magnitude of the T/S (%) in the liver can be determined.

The distribution ratio in the liver to lung tissue after administration may be a distribution ratio at about 40 hours to about 180 hours, about 45 hours to about 170 hours, or about 2 days to about 7 days after administration, but is not limited thereto.

The distribution ratio in the liver to lung tissue after administration may be a distribution ratio of 1: about 2.4 to about 2.7, 1: about 2.4 to about 2.7, 1: about 2.5 to about 2.6, or 1: about 2.5 to 3.7 at 2 days after administration, or about 1: about 2.5 to 3, 1: about 2.7 to about 3, 1: about 2.8 to about 3.0, 1: about 2.8 to about 2.9, or 1: about 2.8 to about 3.9 at about 7 days after administration, but is not limited thereto.

Regarding the distribution ratio in the liver to heart after administration, (a) the distribution ratio in the liver to heart at about 40 hours to about 50 hours after administration may be 1: about 1.6 to about 3.0; and (b) the distribution ratio in the liver to heart at about 160 hours to about 180 hours after administration may be 1: about 2.8 to about 7.0, but is not limited thereto.

Regarding the distribution ratio in the liver to heart after administration, (a) the distribution ratio in the liver to heart at about 40 hours to about 50 hours, about 45 hours to about 50 hours, or about 2 days after administration may be 1: about 1.6 to 3.0, 1: about 1.6 to 2.5, 1: about 1.6 to about 2.2, 1: about 1.7 to about 2.1, 1: about 1.7 to about 2.0, 1: about 1.8 to 2.0, 1: about 1.8 to 2.0, 1: about 1.89 to 2.0, 1: about 1.8 to 2.0, or 1: about 1.8 to 2.9; and (b) the distribution ratio in the liver to heart at about 160 hours to about 180 hours, about 160 hours to about 180 hours, or about 7 days after administration may be 1: about 2.8 to about 4, 1: about 2.9 to about 3.8, 1: about 3.0 to about 3.8, 1: about 3.0 to about 3.6, 1: about 3.0 to about 3.5, 1: about 3.0 to about 3.4, 1: about 3.1 to about 3.4, 1: about 3.2 to about 3.4, 1: about 3.2 to about 3.3, 1: about 2.8 to about 7.0, or 1: about 3.3 to about 6.6, but is not limited thereto.

The distribution ratio may be measured on the basis of T/S (%), and when the T/S (%) in the heart is set to 1 as a reference value, the magnitude of the T/S (%) in the liver can be determined.

The term "about" as used herein refers to a range encompassing ±0.5, ±0.4, ±0.3, ±0.2, ±0.1, ±0.05, and the like, and includes all of the values in the range equivalent or similar to those stated after this term, but is not limited thereto.

The liver-targeted drug may include a substance capable of being targeted to liver tissue.

Specifically, the liver-targeted drug may be a peptide or protein containing glucagon or its derivative capable of inducing targeting to the liver, wherein the peptide or protein includes a peptide sequence having activity to the glucagon receptor. For example, the peptide or protein may be in the form of a peptide including the amino acid sequence of General Formula 1 or a long-acting conjugate including the same, or a peptide including the amino acid sequence of General Formula 2 or a long-acting conjugate including the same, but is not limited thereto. The peptide may have an activity to the glucagon receptor, and specifically may be a glucagon derivative, but is not limited thereto.

In the present invention, glucagon or a derivative thereof, which is a liver-targeted drug, indicates that the corresponding drug retains binding affinity for the glucagon receptor enough to be targeted to the liver from the blood when delivered into the blood of an animal. More specifically, when the distribution ratio of the corresponding drug in an organ is investigated at least 6 hours, at least 30 hours, at least 48 hours, or at least 168 hours after delivery of the drug into the blood of an animal, the distribution ratio of the drug in the liver is at least about 5%, at least about 10%, at least about 15%, at least about 20%, at least about 30%, or at least about 40% higher than that in at least one other organ, such as the heart, lung, large intestine, spleen, pancreas, adipose tissue, small intestine, stomach, muscle, kidney, or brain, but the distribution ratio of the drug is not particularly limited as long as it is higher than those in comparative organs.

The substance having activity to the glucagon receptor includes a variety of substances having a significant level of activity to the glucagon receptor, for example, a substance in the form of a compound or peptide.

Although not particularly limited, the substance having a significant level of activity to the glucagon receptor may be not only native glucagon but also a substance exhibiting *in vitro* activity of 0.1% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, or 100% or more to the glucagon receptor, compared with the native ligand (native glucagon) of the corresponding receptor.

With respect to a method for measuring such *in vitro* activity of glucagon or a glucagon derivative, reference may be made to Experimental Example 1 of the present specification, but the method is not particularly limited thereto.

Examples of the substance having activity to the glucagon receptor may be native glucagon, an agonist thereof, or a derivative thereof, but are not particularly limited thereto.

Examples of the glucagon derivative according to the present invention include a peptide having at least one difference in amino acid sequence compared with native glucagon, a peptide having an alteration in the native glucagon sequence through modification, and a native glucagon mimic capable of activating the glucagon receptor like native glucagon. Any peptide that can show a high distribution in the liver among the organs in the body of a subject administered may fall within the scope of the present invention.

The glucagon derivative may encompass those disclosed in WO 2016/108586 and WO 2017/003191, the entire descriptions of which are incorporated herein by reference. Additionally, a method for preparing a long-acting conjugate of the glucagon derivative peptide is disclosed in WO 2017/003191, the entire description of which is incorporated herein by reference.

Such glucagon derivatives may exhibit improved properties by having a changed pl compared with native glucagon. Additionally, the glucagon derivatives may have improved solubility while retaining the activity to activate the glucagon receptor, but is not limited thereto.

The glucagon derivatives may be non-naturally occurring.

Meanwhile, the native glucagon may include the following amino acid sequence:

His-Ser-Gln-Gly-Thr-Phe-Thr-Ser-Asp-Tyr-Ser-Lys-Tyr-Leu-Asp-Ser-Arg-
Arg-Ala-Gln-Asp-Phe-Val-Gln-Trp-Leu-Met-Asn-Thr (SEQ ID NO: 1).

The term "isoelectric point" or "pl" as used herein refers to a pH value at which a molecule, such as a polypeptide or peptide, has no net charge (0). In a polypeptide with various charged functional groups, a sum of charges is zero at the pl value. The total net charge of a polypeptide will be negative at a pH higher than the pl, and the total net charge of a polypeptide will be positive at a pH lower than the pl.

The pl value may be determined on an immobilized pH gradient gel composed of polyacrylamide, starch, or agarose by isoelectric electrophoresis or may be estimated, for example, from an amino acid sequence using a pl/MW tool (http://expasy.org/tools/pi_tool.html; Gasteiger et al., 2003) in an ExPASy server.

The term "changed pl" as used herein refers to having a pl which is different from that of native glucagon, that is, reduced or increased compared with that of native glucagon, due to the substitution of a part of the amino acid sequence of native glucagon with negatively or positively charged amino acid residues. A peptide having such a changed pl is a glucagon derivative and may exhibit improved solubility and/or high stability at a neutral pH. However, the changed pl is not particularly limited thereto.

For example, the glucagon derivative may have a changed pl value, different from the pl value (6.8) of native glucagon, and the pl value may be lower than 6.8, 6.7 or lower, or 6.5 or lower, and higher than 6.8, 7 or higher, or 7.5 or higher, but is not limited thereto, and any pl value that is different from that of native glucagon falls within the scope of the present invention. In particular, any glucagon derivative falls within the scope of the present invention as long as the glucagon derivative aggregates at a lower level than native glucagon, caused by improved solubility at a neutral pH due to a pl value different from that of native glucagon.

For example, the glucagon derivative may have a pl value of 4 to 6.5 and/or 7 to 9.5, 7.5 to 9.5, or 8.0 to 9.3, but is not limited thereto. In such a case, the glucagon derivative has a higher or lower pl value than native glucagon, and thus can exhibit improved solubility and higher stability at a neutral pH compared with native glucagon. However, the present invention is not limited thereto.

Specifically, a derivative of native glucagon may be obtained through alteration by any one method of substitution, addition, deletion, and modification of some amino acids in native glucagon or any combination thereof.

Examples of the glucagon derivative prepared by a combination of the above-described methods include a peptide including at least one different amino acid residue in the amino acid sequence compared with native glucagon and a deleted amino acid residue at the N-terminus and having a function of activating the glucagon receptor, but are not limited thereto. The derivatives of native glucagon according to the present invention may be prepared by a combination of various methods for the preparation of derivatives.

In addition, such an alteration for the preparation of derivatives of native glucagon includes all of: alterations using L-type or D-type amino acids, and/or a non-native amino acid; and/or alterations by modification of the native sequence, for example, an alteration of a side chain functional group, intramolecular covalent linkage, such as ring formation between side chains, methylation, acylation, ubiquitination, phosphorylation, aminohexanation, or biotinylation. The alteration also includes substitution with a non-native compound.

The alteration also includes all of the additions of one or more amino acids to the amino and/or carboxy terminus of native glucagon.

The amino acids substituted or added may be not only 20 amino acids commonly found in human proteins but also atypical or non-naturally occurring amino acids. Commercial sources of atypical amino acids may include Sigma-Aldrich, ChemPep, and Genzyme Pharmaceuticals. The peptides including these amino acids and typical peptide sequences may be synthesized and purchased from companies specialized in the synthesis of commercial peptides, for example, American Peptide Company and Bachem in USA, or Anygen in Korea.

The amino acid derivatives may also be accessible in a similar manner and, for example, 4-imidazoacetic acid or the like may be used.

Glucagon has a pl value of about 7 and therefore is insoluble in a solution with a physiological pH (pH 4-8) and tends to precipitate at a neutral pH. In an aqueous solution with a pH of 3 or lower, glucagon is dissolved at the initial stage but precipitates within one hour due to gelling. The gelled glucagon mainly consists of β-sheet fibrils, and the administration of the resultant precipitated glucagon via an injection needle or intravenous injection blocks blood vessels, and thus glucagon is not suitable for use as an injection agent. To delay the precipitation process, acidic (pH 2-4) formulations are commonly used, and in such cases, glucagon can be maintained in a relatively non-aggregated state for a short period of time. However, the fibril formation of glucagon is made very rapidly at a low pH, so that such acidic formulations need to be injected upon preparation.

The glucagon derivative of the present invention encompasses those that are developed to have extended action profiles by changing the pl of native glucagon through substitutions of negatively and positively charged amino acid residues. These glucagon derivatives have a changed pl compared with native glucagon and thus can exhibit improved solubility and/or high stability at a neutral pH compared with native glucagon.

In one specific aspect, the glucagon or glucagon derivative may be a peptide including an amino acid sequence of General Formula 1 below:

X1-X2-QGTF-X7-SD-X10-S-X12-X13-X14-X15-X16-X17-X18-X19-X20-X21-F-X23-X24-W-L-X27-X28-X29-X30 (General Formula 1, SEQ ID NO: 46)

wherein,
X1 is histidine, desamino-histidyl, N-dimethyl-histidyl, beta-hydroxy imidazopropionyl, 4-imidazoacetyl, beta-carboxy imidazopropionyl, tryptophan, or tyrosine, or is absent;
X2 is α-methyl-glutamic acid, aminoisobutyric acid (Aib), D-alanine, glycine, N-methylglycine (Sar), serine, or D-serine;
X7 is threonine, valine, or cysteine;
X10 is tyrosine or cysteine;
X12 is lysine or cysteine;
X13 is tyrosine or cysteine;
X14 is leucine or cysteine;
X15 is aspartic acid, glutamic acid, or cysteine;
X16 is glutamic acid, aspartic acid, serine, α-methyl-glutamic acid, or cysteine, or is absent;
X17 is aspartic acid, glutamine, glutamic acid, lysine, arginine, serine, cysteine, or valine, or is absent;
X18 is alanine, aspartic acid, glutamine, glutamic acid, arginine, valine, or cysteine, or is absent;
X19 is alanine, arginine, serine, valine, or cysteine, or is absent;
X20 is lysine, histidine, glutamic acid, glutamine, aspartic acid, arginine, α-methyl-glutamic acid, or cysteine, or is absent;
X21 is aspartic acid, glutamine, leucine, valine, or cysteine, or is absent;
X23 is isoleucine, valine, arginine, or is absent;
X24 is valine, arginine, alanine, cysteine, glutamic acid, lysine, glutamine, α-methyl-glutamic acid, or leucine, or is absent;
X27 is isoleucine, valine, alanine, lysine, methionine, glutamine, or arginine, or is absent;
X28 is glutamine, lysine, asparagine, or arginine, or is absent;
X29 is lysine, alanine, glycine, or threonine, or is absent; and
X30 is cysteine or is absent.

More specifically,
in General Formula 1,
X1 is histidine, tryptophan, or tyrosine, or is absent;
X2 is serine or aminoisobutyric acid (Aib);
X7 is threonine, valine, or cysteine;
X10 is tyrosine or cysteine;
X12 is lysine or cysteine;
X13 is tyrosine or cysteine;
X14 is leucine or cysteine;
X15 is aspartic acid or cysteine;
X16 is glutamic acid, serine, or cysteine;
X17 is aspartic acid, glutamic acid, lysine, arginine, serine, cysteine, or valine;
X18 is aspartic acid, glutamic acid, arginine, or cysteine;
X19 is alanine or cysteine;
X20 is glutamine, aspartic acid, lysine, or cysteine;
X21 is aspartic acid, glutamic acid, leucine, valine, or cysteine;
X23 is isoleucine, valine, or arginine;
X24 is valine, arginine, alanine, glutamic acid, lysine, glutamine, or leucine;
X27 is isoleucine, valine, alanine, methionine, glutamine, or arginine;
X28 is glutamine, lysine, asparagine, or arginine;
X29 is threonine;
X30 is cysteine or is absent.

For example, the peptide may include an amino acid sequence selected from the group consisting of SEQ ID NOS: 1 to 44, specifically, may consist (essentially) of an amino acid sequence selected from the group consisting of SEQ ID NOS: 1 to 44. In another embodiment, the peptide of the present invention may include an amino acid sequence selected from the group consisting of SEQ ID NOS: 2 to 45, specifically, may consist (essentially) of an amino acid sequence selected from the group consisting of SEQ ID NOS: 2 to 45, but is not limited thereto.

Although it is described as "peptide consisting of a specific SEQ ID NO" herein, such expression does not exclude any addition of nonsense sequences upstream or downstream of the amino acid sequence of the corresponding SEQ ID NO or naturally occurring mutations, or silent mutations thereof, as long as the analog has the same or equivalent activity as the analog or the insulin analog consisting of the amino acid sequence of the corresponding SEQ ID NO, and it is obvious that such a sequence addition or mutation also falls within the scope of the present invention. That is, as long as a peptide shows at least a predetermined level of homology or identity despite some differences in the sequence and has a high distribution in the liver among the organs in the human body of a subject administered, such a peptide may fall within the scope of the present invention.

For example, a peptide consisting of a specific SEQ ID NO or a peptide having at least 60% at least 65%, at least 70%, at least 75%, at least 80%, at least 85%, at least 90%, at least 91%, at least 92%, at least 93%, at least 94%, or at least 95% sequence identity with respect to a peptide consisting of a specific SEQ ID NO or a peptide including a specific SEQ ID NO may also be included in the present invention, and if a peptide has a high distribution in the liver among the organs in the body of a subject administered, such a peptide is not delimited to a specific sequence.

The term "homology" or "identity" as used herein refers to a degree of relevance between two given amino acid sequences or nucleotide sequences, and the term may be expressed as a percentage. The terms homology and identity may be often used interchangeably with each other.

Whether any two peptide sequences have homology, similarity, or identity may be determined using any computer algorithm known in the art, such as the "FASTA" program, using default parameters disclosed as in Pearson et al (1988) [Proc. Natl. Acad. Sci. USA 85]: 2444. Alternatively, this may be determined using the Needleman-Wunsch algorithm (Needleman and Wunsch, 1970, J. Mol. Biol. 48: 443-453), which is performed in the Needleman program of the European Molecular Biology Open Software Suite (EMBOSS) package (Rice et al., 2000, Trends Genet. 16: 276-277) (version 5.0.0 or versions thereafter) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.] (1988) SIAM J Applied Math 48: 1073). For example, the homology, similarity, or identity may be determined using BLAST of the National Center for Biotechnology Information database, or ClustalW

The homology, similarity, or identity between peptides may be determined by comparing sequence information using the GAP computer program, for example, Needleman et al., (1970), J Mol Biol. 48:443, as known in Smith and Waterman, Adv. Appl. Math (1981) 2:482. Briefly, the GAP program defines the homology, similarity, or identity as the value obtained by dividing the number of similarly aligned symbols (i.e., amino acids) by the total number of the symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358, 1979 (or the EDNAFULL (EMBOSS version of NCBI NUC4.4) substitution matrix); (2) a penalty of 3.0 for each gap and an additional 0.10 penalty for each symbol in each gap (or a gap opening penalty of 10 and a gap extension penalty of 0.5); and (3) no penalty for end gaps. Therefore, the term "homology" or "identity" as used herein represents the relevance between sequences.

The above description can be applied to other embodiments or aspects of the present invention, but is not limited thereto.

As still another example, the peptide may include an amino acid sequence selected from the group consisting of SEQ ID NOS: 3, 11 to 17, 19 to 27, 29, 31, 33, and 35 to 44 and, specifically, may consist (essentially) of an amino acid sequence selected from the group consisting of SEQ ID NOS: 3, 11 to 17, 19 to 27, 29, 31, 33, and 35 to 44, but is not limited thereto.

As still another example, the peptide may include an amino acid sequence selected from the group consisting of SEQ ID NOS: 12, 14 to 17, 19 to 25, 27, 29, 33, 35 to 38, 40 to 42, and 44 and, specifically, may consist (essentially) of an amino acid sequence selected from the group consisting of SEQ ID NOS: 12, 14, 17, 19 to 25, 27, 29, 33, 35 to 38, 40 to 42, and 44, but is not limited thereto.

As still another example, the peptide may include an amino acid sequence selected from the group consisting of SEQ ID NOS: 2 to 13, 15, 17, 20 to 24, 26 to 30, and 32 to 44 and, specifically, may consist (essentially) of an amino acid sequence selected from the group consisting of SEQ ID NOS: 2 to 13, 15, 17, 20 to 24, 26 to 30, and 32 to 44, but is not limited thereto.

As still another example, the peptide may include an amino acid sequence selected from the group consisting of SEQ ID NOS: 20, 22, 23, 27, 33, 35, 37, 38, 40, 41, 42, and 44 and, specifically, may consist (essentially) of an amino acid sequence selected from the group consisting of SEQ ID NOS: 20, 22, 23, 27, 33, 35, 37, 38, 40, 41, 42, and 44, but is not limited thereto.

As still another example, the peptide may include an amino acid sequence selected from the group consisting of SEQ ID NOS: 20, 22, 23, 27, 33, 37, 38, and 44 and, specifically, may consist (essentially) of an amino acid sequence selected from the group consisting of SEQ ID NOS: 20, 22, 23, 27, 33, 37, 38, and 44, but is not limited thereto.

As still another example, the peptide may include an amino acid sequence selected from the group consisting of SEQ ID NOS: 14, 16, 18, 19, 25, and 31 and, specifically, may consist (essentially) of an amino acid sequence selected from the group consisting of SEQ ID NOS: 14, 16, 18, 19, 25, and 31, but is not limited thereto.

In an embodiment, in a pharmaceutical composition containing a liver-targeted drug and having a high distribution of the drug in the liver among the organs in the body of a subject administered, the liver-targeted drug may be a peptide including an amino acid sequence of General Formula 2 below:

Y-Aib-QGTF-X7-SD-X10-S-X12-Y-L-X15-X16-X17-R-A-X20-X21-F-V-X24-W-L-M-N-T-X30 (General Formula 2, SEQ ID NO: 47)

wherein, in General Formula 2 above,
X7 is threonine (T), valine (V), or cysteine (C);
X10 is tyrosine (Y) or cysteine (C);
X12 is lysine (K) or cysteine (C);
X15 is aspartic acid (D) or cysteine (C);
X16 is glutamic acid (E) or serine (S);
X17 is lysine (K) or arginine (R);
X20 is glutamine (Q) or lysine (K);
X21 is aspartic acid (D) or glutamic acid (E);
X24 is valine (V) or glutamine (Q); and
X30 is cysteine (C) or is absent
(with the proviso that an amino acid sequence of General Formula 2 identical to SEQ ID NO: 12 is excluded).

For example, the peptide may include an amino acid sequence selected from the group consisting of SEQ ID NOS: 13, 15, 19, 33, and 36 to 45 and, specifically, may consist (essentially) of an amino acid sequence selected from the group consisting of SEQ ID NOS: 13, 15, 19, 33, and 36 to 45, but is not limited thereto.

Specifically, in General Formula 2 above,
X7 is threonine, valine, or cysteine;
X10 is tyrosine or cysteine;
X12 is lysine;
X15 is aspartic acid;
X16 is glutamic acid or cysteine;
X17 is lysine or arginine;
X20 is glutamine or lysine;
X21 is aspartic acid or glutamic acid;
X24 is glutamine;
X30 is cysteine, or may be absent, but the peptide is not particularly limited thereto.

For example, the peptide may include an amino acid sequence selected from the group consisting of SEQ ID NOS: 33 and 36 to 44, and specifically, may consist (essentially) of an amino acid sequence selected from the group consisting of SEQ ID NOS: 33 and 36 to 44, but is not limited thereto.

The above-described glucagon may include an intramolecular bridge (e.g., covalent crosslinkage or non-covalent crosslinkage), and specifically, may be in a form that includes a ring. For example, the intramolecular bridge may be in the form where a ring is formed between the 16th and 20th amino acids of the glucagon derivative, but it is not particularly limited thereto.

Non-limiting examples of the ring may include a lactam bridge (or lactam ring).

Additionally, examples of the glucagon derivative include all of those that are modified to include amino acids capable of forming a ring at a desired site so as to include a ring.

Such a ring may be formed between side chains of amino acids within the glucagon derivative, and an example thereof may be a form in which a lactam ring is formed between a side chain of lysine and a side chain of a glutamic acid, but is not particularly limited thereto.

For example, the peptide including an amino acid sequence of General Formula 1 or 2 may be one where each amino acid in each amino acid pair among the amino acid pairs of X10 and X14, X12 and X16, X16 and X20, X17 and X21, X20 and X24, and X24 and X28 in General Formula 1 or 2 is substituted with glutamic acid or lysine, but is not limited thereto. In Xn (n is a natural number), n represents the locus of an amino acid from the N-terminus of the amino acid sequence.

The peptide including an amino acid sequence of General Formula 1 or 2 may be one where each amino acid of an amino acid pair of X12 and X16, X16 and X20, or X17 and X21 is substituted with glutamic acid or lysine, but is not limited thereto.

Also, in General Formula 1 or 2 above, a ring (e.g., lactam ring) may be formed between amino acids of each amino acid pair among one or more amino acid pairs of X10 and X14, X12 and X16, X16 and X20, X17 and X21, X20 and X24, and X24 and X28, but is not limited thereto.

Also, in General Formula 1 or 2 above, X16 may be glutamic acid and X20 may be lysine, and the side chains of X16 and X20 may form a lactam ring, but is not limited thereto.

The peptide may have an increased in-vivo half-life compared with native glucagon, but is not particularly limited thereto.

In addition, the peptide according to the present invention may be in the form where the N-terminus and/or C-terminus is not modified, but a form where the N-terminus and/or C-terminus of the peptide is chemically modified or protected by organic groups, or an amino acid is added to a terminus of the peptide for its protection from proteases *in vivo* while increasing its stability, may also fall within the scope of the peptides of the present invention. When the C-terminus is not modified, the terminus of the peptide according to the present invention may have a carboxy group, but is not particularly limited thereto.

Alternatively, a chemically synthesized peptide has electrically charged N- and C-termini, and thus for the elimination of these charges, the N-terminus may be acetylated and/or the C-terminus may be amidated, but is not particularly limited thereto.

Unless specified otherwise herein, the statements in the detailed description or claims with respect to the "peptide" according to the present invention or a "conjugate" in which such a peptide is covalently linked to a biocompatible material, may be applied to the ranges including all of not only the corresponding peptide or conjugate but also salts of the corresponding peptide or conjugate (e.g., pharmaceutically acceptable salts of the peptide) or solvates thereof. Therefore, although it is described as only "peptide" or "conjugate" herein, the corresponding description are equally applied to a specific salt thereof, a specific solvate thereof, and a specific solvate of the specific salt thereof. These salts may be, for example, in the form where any pharmaceutically acceptable salt is used.

The kind of salt is not particularly limited. However, the salt is preferably in the form that is safe and effective to a subject, for example, a mammal, but is not particularly limited thereto.

The term "pharmaceutically acceptable" refers to a substance that can be effectively used for a desired purpose without causing excessive toxicity, irritation, allergic responses, and the like within the scope of the medical and pharmaceutical decision.

The term "pharmaceutically acceptable salt" as used herein refers to a salt derived from pharmaceutically acceptable inorganic acids, organic acids, or bases. Examples of suitable acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, and the like. Salts derived from suitable bases may include alkali metals such as sodium and potassium, alkali earth metals such as magnesium, ammonium, and the like.

The term "solvate" as used herein refers to a complex formed of the peptide, conjugate, or salt thereof according to the present invention and a solvent molecule.

The peptide according to the present invention may include a peptide, of which the C-terminus is amidated or has a free carboxy (-COOH) group, or a peptide, of which the C-terminus is not modified, but is not limited thereto.

In an embodiment, the C-terminus of the peptide may be amidated, but is not limited thereto.

In an embodiment, the peptide may be aglycosylated, but is not limited thereto.

The peptide of the present invention may be synthesized through solid-phase synthesis, produced by a recombinant method, or prepared by a commercial order, but is not limited thereto.

In addition, the peptide of the present invention may be synthesized by a method well known in the art, for example, an automatic peptide synthesizer, according to the length thereof, or may be produced by genetic engineering technology.

Specifically, the peptide of the present invention may be prepared by a standard synthesis method, a recombinant expression system, or any other method known in the art. Hence, the peptide according to the present invention may be synthesized by a number of methods including, for example, the following:
(a) a method where a peptide is synthesized by a solid-phase or liquid-phase process stepwise or by fragment assembly, and a final peptide product is separated and purified;
(b) a method where a nucleic acid construct encoding a peptide is expressed in a host cell and an expression product is collected from a host cell culture;
(c) a method where the *in vitro* cell-free expression of a nucleic acid construct encoding a peptide is performed and an expression product is collected therefrom; or
a method where peptide fragments are obtained by any combination of (a), (b), and (c), the fragments are linked to obtain a peptide, and then the corresponding peptide is collected.

Additionally, the peptide including an amino acid sequence of General Formula 1 or 2, or a glucagon derivative may be in the form of a long-acting conjugate in which the peptide or glucagon derivative is linked to a biocompatible substance portion capable of increasing *in vivo* half-life of the peptide or glucagon derivative, but is not limited thereto. Herein, the biocompatible material portion may be used interchangeably with a carrier.

The peptide (conjugate form) in the form of a long-acting conjugate, including an amino acid sequence of General Formula 1 or 2, or the peptide itself, including an amino acid sequence of General Formula 1 or 2, is a liver-targeted drug and is an active ingredient of a pharmaceutical composition having a high distribution of the drug in the liver among the organs in the body of a subject to whom it is administered.

The term "long-acting conjugate" as used herein refers to a form in which a biocompatible material portion or carrier is linked to a biologically active substance (e.g., a glucagon derivative), and specifically, the conjugate includes a peptide moiety and a biocompatible material portion covalently linked to the peptide moiety, wherein the peptide moiety may include an amino acid sequence of General Formula 1 or 2, or sequences that are the same as SEQ ID NOS: 2 to 11 and 13 to 45 or includes the same. In the long-acting conjugate, the biocompatible material portion or carrier may be covalently linked to a biologically active substance, but is not particularly limited thereto.

In the present invention, the conjugate of the peptide can exhibit an increased duration of efficacy and/or an increase in blood half-life, compared with a peptide to which a carrier is not linked, and in the present invention, such a conjugate is referred to as a "long-acting conjugate".

Such a conjugate may be a non-naturally occurring conjugate.

In a specific embodiment of the present invention, the long-acting conjugate refers to a form in which an immunoglobulin Fc region is linked to a glucagon derivative. Specifically, the conjugate may be a conjugate in which an immunoglobulin Fc region is covalently linked to a glucagon derivative via a linker, but is not particularly limited thereto.

In an embodiment, the immunoglobulin Fc region and X may not be glycosylated, but are not limited thereto.

In an embodiment of the present invention, the long-acting conjugate is represented by chemical formula 1:

[Chemical Formula 1] X-L-F

wherein,
X represents the peptide;
L represents a linker containing ethylene glycol repeating units;
F represents an immunoglobulin Fc region; and
the "-" symbols represent covalent linkages between X and L and between L and F, respectively.

X in the long-acting conjugate of Chemical Formula 1 may be the glucagon derivative as described above, but is not limited thereto.

In an embodiment, X in the long-acting conjugate of Chemical Formula 1 may be the peptide including an amino acid sequence of General Formula 2 above.

Specifically, X may be a peptide including an amino acid sequence of any one selected from the group consisting of SEQ ID NOS: 13, 15, 19, 33, and 36 to 45, or a peptide including an amino acid sequence of any one selected from the group consisting of SEQ ID NOS: 33 and 36 to 44, but is not limited thereto.

As another example, X may be a peptide including an amino acid sequence of General Formula 1, or X may be a peptide including the amino acid sequence of any one of SEQ ID NOS: 2 to 45, or a peptide including the amino acid sequence of any one of SEQ ID NOS: 2 to 11 and 13 to 45, or X may be a peptide including an amino acid sequence selected from the group consisting of SEQ ID NOS: 20, 22, 23, 27, 33, 35, 37, 38, 40, 41, 42, and 44, but is not limited thereto.

F in the conjugate is X, that is, a liver-targeted drug, specifically, a peptide having activity to the glucagon receptor, and corresponds to one element of moieties constituting the conjugate. All of the above descriptions are applied to the liver-targeted drug. F may be linked to X by a covalent chemical linkage or non-covalent chemical linkage, or F and X may be linked to each other through L by a covalent chemical linkage, a non-covalent chemical linkage, or a combination thereof.

The term "long-acting conjugate of Chemical Formula 1" as used herein refers to a form in which a glucagon derivative and an immunoglobulin Fc region are linked to each other via a linker, and the conjugate may exhibit an increased duration of efficacy compared with a glucagon derivative to which an immunoglobulin Fc region is not linked.

In the long-acting conjugate, F is a substance capable of increasing the half-life of X, i.e., a glucagon derivative, and corresponds to one element of moieties constituting the conjugate of the present invention.

In the long-acting conjugate of Chemical Formula 1, the linkage between X, the peptide, which is the glucagon derivative, and the immunoglobulin Fc region may be a physical or chemical linkage, or a non-covalent or covalent linkage, and specifically a covalent linkage, but is not limited thereto.

Additionally, a method of linking X, which is a glucagon derivative, and the immunoglobulin Fc region of the long-acting conjugate of Chemical Formula 1 is not particularly limited, but the glucagon derivative and the immunoglobulin Fc region may be linked to each other via a linker.

In Chemical Formula 1 above, X and F may be linked to each other via L by a covalent linkage.

More specifically, X and L, and L and F may be linked to each other by a covalent linkage, and in particular, the conjugate may be a conjugate in which X, L, and F are linked by a covalent linkage in the order of Chemical Formula 1.

In addition, X may be linked to F via a linker (L).

Meanwhile, L may be a non-peptide linker, for example, a linker containing ethylene glycol repeating units.

In the present invention, the "non-peptide linker" includes a biocompatible polymer having two or more repeating units linked to each other. The repeating units are linked to each other by any covalent linkage but not a peptide linkage. The non-peptide linker may be one element of moieties constituting the conjugate of the present invention, and corresponds to L in Formula 1. As the non-peptide linker usable in the present invention, any polymer that has resistance to *in vivo* protease may be used without limitation. In the present invention, the non-peptide linker may be used interchangeably with a non-peptide polymer.

Although not particularly limited, the non-peptide linker may be a linker containing ethylene glycol repeating units, and for example, may be polyethylene glycol, and derivatives thereof already known in the art and derivatives that can be easily prepared within the level of skill in the art also fall within the scope of the present invention.

The repeating units of the non-peptide linker may be ethylene glycol repeating units, and specifically, the non-peptide linker may include, at the ends thereof, functional groups used in the preparation of the conjugate before being configured into the conjugate, while including ethylene glycol repeating units. The long-acting conjugate according to the present invention may be in the form in which X and F are linked through the functional groups, but is not limited thereto. In the present invention, the non-peptide linker may include two, or three or more functional groups, and the respective functional groups may be identical to or different from each other, but is not limited thereto.

Specifically, the linker may be polyethylene glycol (PEG) represented by Chemical Formula 2 below, but is not limited thereto: 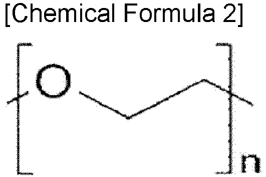 wherein, n represents 10 to 2,400, n represents 10 to 480, or n represents 50 to 250, but is not limited thereto.

In the long-acting conjugate, the PEG moiety may include not only the - (CH₂CH₂O)ₙ- structure, but also an oxygen atom interposed between a linking element and -(CH₂CH₂O)ₙ-, but is not limited thereto.

In an embodiment, the ethylene glycol repeating units may be represented by, for example, [OCH₂CH₂]n, where, the value of n is a natural number, and the average molecular weight, for example, the number average molecular weight, of the [OCH₂CH₂]n region in the peptide conjugate may be determined to be larger than 0 kDa up to about 100 kDa, but is not limited thereto. As another example, the value of n is a natural number, and the average molecular weight, for example, the number average molecular weight of the [OCH₂CH₂]n region in the peptide conjugate may be about 1 to about 100 kDa, about 1 to about 80 kDa, about 1 to about 50 kDa, about 1 to about 30 kDa, about 1 to about 25 kDa, about 1 to about 20 kDa, about 1 to about 15 kDa, about 1 to about 13 kDa, about 1 to about 11 kDa, about 1 to about 10 kDa, about 1 to about 8 kDa, about 1 to about 5 kDa, about 1 to about 3.4 kDa, about 3 to about 30 kDa, about 3 to about 27 kDa, about 3 to about 25 kDa, about 3 to about 22 kDa, about 3 to about 20 kDa, about 3 to about 18 kDa, about 3 to about 16 kDa, about 3 to about 15 kDa, about 3 to about 13 kDa, about 3 to about 11 kDa, about 3 to about 10 kDa, about 3 to about 8 kDa, about 3 to about 5 kDa, about 3 to about 3.4 kDa, about 8 to about 30 kDa, about 8 to about 27 kDa, about 8 to about 25 kDa, about 8 to about 22 kDa, about 8 to about 20 kDa, about 8 to about 18 kDa, about 8 to about 16 kDa, about 8 to about 15 kDa, about 8 to about 13 kDa, about 8 to about 11 kDa, about 8 to about 10 kDa, about 9 to about 15 kDa, about 9 to about 14 kDa, about 9 to about 13 kDa, about 9 to about 12 kDa, about 9 to about 11 kDa, about 9.5 to about 10.5 kDa, or about 10 kDa, but is not limited thereto.

In a specific embodiment, the conjugate of Chemical Formula 2 may have a structure in which an immunoglobulin region (F) is linked to a peptide (X) including an amino acid sequence of General Formula 1 by a covalent linkage via a linker containing ethylene glycol repeating units, but is not limited thereto.

The polyethylene glycol is a general term encompassing all of the forms of ethylene glycol homopolymers, PEG copolymers, or monomethyl-substituted PEG polymers (mPEG), but is not particularly limited thereto.

The molecular weight of the non-peptide polymer may be in the range of 1 kDa to 100 kDa, specifically 1 kDa to 20 kDa, or 1 kDa to 10 kDa, but is not limited thereto. Additionally, the non-peptide linker of the present invention, which is linked to the polypeptide corresponding to F, may employ not only a single kind of polymer but also a combination of different kinds of polymers.

In a specific embodiment, both ends of the non-peptide linker may be linked to a thiol group, an amino group, or a hydroxyl group of the immunoglobulin Fc region and a thiol group, an amino group, an azide group, or a hydroxyl group of the peptide (X), respectively, but are not limited thereto.

Specifically, the linker may include, at both ends thereof, reactive groups capable of binding to the immunoglobulin Fc region and the peptide (X), respectively. Specifically, reactive groups capable of binding to a thiol group of cysteine; an amino group located at the N-terminus, lysine, arginine, glutamine, and/or histidine; and/or a hydroxyl group located at the C-terminus in the immunoglobulin Fc region, and to a thiol group of cysteine; an amino group of lysine, arginine, glutamine, and/or histidine; an azide group of azido-lysine; and/or a hydroxyl group in the peptide (X), but is not limited thereto.

More specifically, the reactive groups of the linker may be one or more selected from the group consisting of an aldehyde group, a maleimide group, and a succinimide derivative, but are not limited thereto.

In the above, an example of the aldehyde group may be a propionaldehyde group or butyl aldehyde group, but is not limited thereto.

In the above, an example of a succinimide derivative may be succinimidyl valerate, succinimidyl methylbutanoate, succinimidyl methylpropionate, succinimidyl butanoate, succinimidyl propionate, N-hydroxysuccinimide, hydroxy succinimidyl, succinimidyl carboxymethyl, or succinimidyl carbonate, but is not limited thereto.

The linker may be linked to F, the immunoglobulin Fc region, and X, the peptide (glucagon derivative), through the reactive groups, to be converted to a linker moiety.

Additionally, a final product produced through reductive amination (or reductive alkylation) by an aldehyde linkage is significantly more stable than that produced by an amide linkage. The aldehyde reactive group selectively reacts at the N-terminus at a low pH, while the aldehyde reactive group may form a covalent linkage with a lysine residue at a high pH, for example, pH 9.0, but is not limited thereto.

The reactive groups at both of the ends of the linker of the present invention may be the same as or different from each other. The linker may have aldehyde reactive groups at both ends. Alternatively, the linker may have an aldehyde group and a maleimide group at the ends, or may have an aldehyde group and a succinimide reactive group at the ends. However, the linker is not limited as long as F, specifically, the immunoglobulin Fc region, and X can be linked to either end of the linker.

For example, the linker may have a maleimide group at one end and an aldehyde group, a propionaldehyde group, or a butyraldehyde group at the other end. As another example, the linker may have a succinimidyl group at one end and a propionaldehyde group or a butyraldehyde group at the other end.

When polyethylene glycol having a hydroxy reactive group at propionaldehyde end is used as a linker, the conjugate of the present invention may be prepared by activating the hydroxy group to various reactive groups by known chemical reactions or by using commercially available polyethylene glycol having a modified reactive group.

In a specific embodiment, the reactive group of the linker may be linked to a cysteine residue of the peptide (X), more specifically to the -SH group of cysteine, but the linker is not limited thereto.

When maleimide-PEG-aldehyde is used, the maleimide group may be linked to the -SH group of the peptide by a thioether linkage, and the aldehyde group may be linked to the -NH₂ group of the immunoglobulin Fc region through reductive alkylation, but is not limited thereto, and such a case merely corresponds to one example.

Through such reductive alkylation, a structure such as -PEG-O-CH₂CH₂CH₂NH-immunoglobulin Fc may be formed by linking an amino group at the N-terminus of an immunoglobulin Fc region to an oxygen atom located at one end of PEG through a linker reactive group having a structure of -CH₂CH₂CH₂-, and a structure where one end of the PEG is linked to a sulfur atom located at a cysteine of the peptide through a thioether linkage may be formed. The above-described thioether linkage may contain a structure of

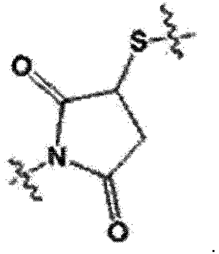

However, the linker is not particularly limited to the above example, and such as case merely corresponds to one example.

In the conjugate, the reactive group of the linker may be linked to -NH₂ located at the N-terminus of the immunoglobulin Fc region, but such a case merely corresponds to one example.

In the conjugate, the peptide according to the present invention may be linked to a linker having a reactive group through the C-terminus thereof, but such a case merely corresponds to one example.

In the present invention, the term "C-terminus" refers to the carboxy terminus of a peptide, and for the purpose of the present invention, the term refers to a position at which the peptide can be linked to the linker. For example, the C-terminus may include the amino acid residue at the very end of the C-terminus as well as surrounding amino acid residues near the C-terminus, and specifically may include the 1st to 20th amino acid residues from the very end, but the C-terminus is not limited thereto.

In an embodiment, the conjugate of Chemical Formula 1 may have a structure of Chemical Formula 3 below: 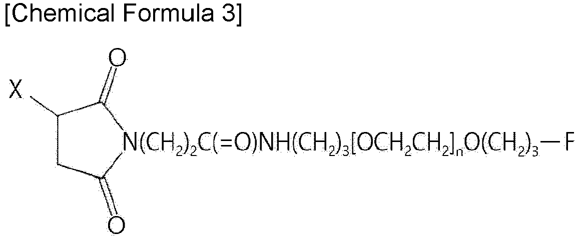
wherein, in Chemical Formula 3, X represents the peptide (glucagon derivative) described above;
F represents a human immunoglobulin Fc region; and
n represents a natural number. Particularly, n is as described above.

In an embodiment, the long-acting conjugate of Chemical Formula 3 has a structure in which the peptide X and the immunoglobulin Fc region F are covalently linked through an ethylene glycol repeating unit region interposed therebetween, wherein X may be linked to a succinimide ring in Chemical Formula 3, and F may be linked to an oxypropylene group in Chemical Formula 3.

In Chemical Formula 3 above, the value of n may be determined such that the average molecular weight, for example, the number average molecular weight, of the [OCH₂CH₂]n region in the peptide conjugate is 1 to 100 kDa or 1 to 20 kDa or 10 kDa, but is not limited thereto.

In an embodiment, the site of the succinimide ring of Chemical Formula 3, to which X is linked, may be a sulfur atom of the C-terminal cysteine of X.

A site of F, which is linked to the oxypropylene group, is not specifically limited. In an embodiment of the present invention, the site of F, which is linked to the oxypropylene group, may be an N-terminal nitrogen or a nitrogen atom (e.g., epsilon nitrogen of lysine) of a residue inside F. In one specific embodiment of the present invention, the site of F, which is linked to the oxypropylene group, may be an N-terminal proline of F, but is not limited thereto.

In the conjugate, the reactive group of the non-peptide polymer may be linked to -NH₂ located at the N-terminus of the immunoglobulin Fc region, but such a case merely corresponds to one example.

As a specific example, F in Chemical Formula 1 is an immunoglobulin Fc region. For example, the immunoglobulin Fc region may be derived from IgG, but is not particularly limited thereto.

The term "immunoglobulin Fc region" as used herein refers to a region including heavy chain constant region 2 (CH2) and/or heavy chain constant region 3 (CH3), excluding the heavy chain and light chain variable regions of an immunoglobulin. The immunoglobulin Fc region may be one element of moieties constituting the long-acting conjugate of the present invention. The immunoglobulin Fc region may be used interchangeably with the term "immunoglobulin Fc fragment".

Herein, the Fc region encompasses not only a native sequence obtained by papain digestion of an immunoglobulin, but also derivatives thereof, for example, variants, of which the sequences are different from the native sequence by conversion of one or more amino acid residues in the native sequence by deletion, insertion, non-conservative or conservative substitution, or a combination thereof. The derivatives, substituents, and variants are assumed to possess the ability to bind to FcRn. In the present invention, F may be a human immunoglobulin region, but is not limited thereto. F may have a structure where two polypeptide chains are linked by a disulfide linkage, or a structure where two polypeptide chains are linked through only a nitrogen atom of one of the two chains, but is not limited thereto. The linkage through the nitrogen atom may be made on the epsilon amino group of lysine or the N-terminus amino group by reductive amination.

The reductive amination refers to a reaction in which an amine group or an amino group of one reactant reacts with an aldehyde (i.e., a functional group capable of reductive amination) of another reactant to form an amine and then form an amine linkage by reductive reaction, and this is an organic synthetic reaction widely known in the art.

In an embodiment, F may be linked through a nitrogen atom of the N-terminus proline thereof, but is not limited thereto.

This immunoglobulin Fc region may include a hinge region in the heavy chain constant region, but is not limited thereto.

In the present invention, the immunoglobulin Fc region may include a specific hinge sequence in the N-terminus.

The term "hinge sequence" as used herein refers to a region which is located in the heavy chain and forms a dimer of the immunoglobulin Fc region through an inter-disulfide bond.

In the present invention, the hinge sequence may be modified such that a part of the hinge sequence having the following amino acid sequence is deleted to have only one cysteine residue, but is not limited thereto.

Glu-Ser-Lys-Tyr-Gly-Pro-Pro-Cys-Pro-Ser-Cys-Pro (SEQ ID NO: 50).

The hinge sequence may include only one cysteine residue by the deletion of the 8th or 11th cysteine residue in the hinge sequence of SEQ ID NO: 50. The hinge sequence of the present invention may consist of 3 to 12 amino acids, including only one cysteine residue, but is not limited thereto. More specifically, the hinge sequence of the present invention may have the following sequence: Glu-Ser-Lys-Tyr-Gly-Pro-Pro-Pro-Ser-Cys-Pro (SEQ ID NO: 51), Glu-Ser-Lys-Tyr-Gly-Pro-Pro-Cys-Pro-Ser-Pro (SEQ ID NO: 52), Glu-Ser-Lys-Tyr-Gly-Pro-Pro-Cys-Pro-Ser (SEQ ID NO: 53), Glu-Ser-Lys-Tyr-Gly-Pro-Pro-Cys-Pro-Pro (SEQ ID NO: 54), Lys-Tyr-Gly-Pro-Pro-Cys-Pro-Ser (SEQ ID NO: 55), Glu-Ser-Lys-Tyr-Gly-Pro-Pro-Cys (SEQ ID NO: 56), Glu-Lys-Tyr-Gly-Pro-Pro-Cys (SEQ ID NO: 57), Glu-Ser-Pro-Ser-Cys-Pro (SEQ ID NO: 58), Glu-Pro-Ser-Cys-Pro (SEQ ID NO: 59), Pro-Ser-Cys-Pro (SEQ ID NO: 60), Glu-Ser-Lys-Tyr-Gly-Pro-Pro-Ser-Cys-Pro (SEQ ID NO: 61), Lys-Tyr-Gly-Pro-Pro-Pro-Ser-Cys-Pro (SEQ ID NO: 62), Glu-Ser-Lys-Tyr-Gly-Pro-Ser-Cys-Pro (SEQ ID NO: 63), Glu-Ser-Lys-Tyr-Gly-Pro-Pro-Cys (SEQ ID NO: 64), Lys-Tyr-Gly-Pro-Pro-Cys-Pro (SEQ ID NO: 65), Glu-Ser-Lys-Pro-Ser-Cys-Pro (SEQ ID NO: 66), Glu-Ser-Pro-Ser-Cys-Pro (SEQ ID NO: 67), Glu-Pro-Ser-Cys (SEQ ID NO: 68), and Ser-Cys-Pro (SEQ ID NO: 69).

More specifically, the hinge sequence may include the amino acid sequence of SEQ ID NO: 60 (Pro-Ser-Cys-Pro) or SEQ ID NO: 69 (Ser-Cys-Pro), but is not limited thereto.

The immunoglobulin Fc region of the present invention may be in the form in which two molecules of the immunoglobulin Fc chain form a dimer due to the presence of a hinge sequence, and the conjugate of Chemical Formula 1 of the present invention may be in the form in which one end of the linker is linked to one chain of the dimeric immunoglobulin Fc region, but is not limited thereto.

The term "N-terminus" as used herein refers to the amino terminus of a protein or polypeptide, and the N-terminus may include the very end of the amino terminus, or 1, 2, 3, 4, 5, 6, 7, 8, 9, or 10 or more amino acids from the very end of the amino terminus. The immunoglobulin Fc region of the present invention may include a hinge sequence in the N-terminus, but is not limited thereto.

In addition, the immunoglobulin Fc region of the present invention may be an extended Fc region, which includes a part or the entirety of heavy chain constant region 1 (CH1) and/or light chain constant region 1 (CL1), excluding only the heavy chain and light chain variable regions of an immunoglobulin, as long as it has substantially the same or an improved effect compared with its native form. Additionally, the immunoglobulin Fc region of the present invention may be a region in which a significantly long amino acid sequence portion corresponding to CH2 and/or CH3 is removed.

For example, the immunoglobulin Fc region of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination of one or two or more domains among a CH1 domain, a CH2 domain, a CH3 domain, and a CH4 domain and an immunoglobulin hinge region (or a part of the hinge region), (e.g., a combination of CH2 and CH3 domains and a high region or a part thereof, and a dimeric form of two polypeptides having the above-described combination); and 6) a dimer of each domain of the heavy chain constant region and a light chain constant region, but the immunoglobulin Fc region is not limited thereto.

In the present invention, the immunoglobulin Fc region may be in the form of a dimer or multi-mer, consisting of single-chain immunoglobulins consisting of domains of the same origin, but is not limited thereto.

In an embodiment, the immunoglobulin Fc region may be in a dimeric form, and one molecule of X may be covalently linked to one Fc region of the dimeric form, and particularly, the immunoglobulin Fc and X may be covalently linked to each other through a non-peptide polymer. Meanwhile, two molecules of X may be also symmetrically conjugated to one Fc region of the dimeric form. The immunoglobulin Fc and X may be linked to each other through a non-peptide linker. However, the present invention is not limited to the above-described embodiments.

The immunoglobulin Fc region of the present invention includes not only the native amino acid sequence and derivatives thereof. The amino acid sequence derivative means an amino acid sequence which is different from the native amino acid sequence by the deletion, insertion, or a non-conservative or conservative substitution of at least one amino acid residue, or a combination thereof in the native amino acid sequence.

For example, the amino acid residues at positions 214 to 238, 297 to 299, 318 to 322, or 327 to 331 in IgG Fc, which are known to be important for linkage, may be used as sites suitable for alteration.

In addition, various types of derivatives are possible by, for example, removing a site capable of forming a disulfide bond, deleting some amino acid residues at the N-terminus of native Fc, or adding a methionine residue at the N-terminus of native Fc. In addition, a complement binding site, for example, a C1q-binding site, may be removed, and an antibody dependent cell mediated cytotoxicity (ADCC) site may be removed to remove effector functions. The techniques for preparing such sequence derivatives of the immunoglobulin Fc region are disclosed in WO 97/34631 and WO 96/32478, and the like.

Amino acid exchanges in proteins and peptides that do not alter the entire activity of molecules are well known in the art (H. Neurath, R. L. Hill, The Proteins, Academic Press, New York, 1979). The exchanges that occur most commonly are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, or the like.

The above-described Fc derivatives exhibit a biological activity equivalent to that of the Fc region of the present invention, and may have an enhanced structural stability of the Fc region against heat, pH, and the like.

Such an Fc region may be obtained from native forms isolated from living bodies of humans or animals, such as cows, goats, pigs, mice, rabbits, hamsters, rats, and guinea pigs, or may be recombinant forms or derivatives thereof obtained from transformant animal cells or microorganisms. Particularly, the Fc region may be obtained from a native form by isolating a whole immunoglobulin from a living human or animal body and then treating the isolated immunoglobulin with protease. The isolated whole immunoglobulin is digested into Fab and Fc when treated with papain, and digested into pF'c and F(ab)₂ when treated with pepsin. These may be subjected to size-exclusion chromatography or the like to separate Fc or pF'c therefrom. In a more specific embodiment, the immunoglobulin Fc region is a recombinant immunoglobulin Fc region where a human-derived Fc region is obtained from a microorganism.

Alternatively, the immunoglobulin Fc region may be in the form of native glycans, increased glycans compared with the native form, or decreased glycans compared with the native form, or may be in a deglycosylated form. The increase, decrease, or removal of the immunoglobulin Fc glycans may be achieved by using conventional methods, such as a chemical method, an enzymatic method, and a genetic engineering method using a microorganism. Particularly, the immunoglobulin Fc region obtained by removal of glycans from Fc exhibits a significant deterioration in binding affinity for the complement c1q and a reduction or loss in antibody-dependent cytotoxicity or complement-dependent cytotoxicity, and thus causes no unnecessary immune response in vivo. In this regard, an immunoglobulin Fc region in a deglycosylated or aglycosylated form may be more suitable to meet the original purpose of the present invention as a drug carrier.

The term "deglycosylation" as used herein indicates an Fc region in which glycans are removed by an enzyme, and the term aglycosylation means a non-glycosylated Fc region produced in prokaryotes, more specifically *E*. *coli.*

Meanwhile, the immunoglobulin Fc region may be originated from humans, or animals, such as cows, goats, pigs, mice, rabbits, hamsters, rats, and guinea pigs, and in a more specific embodiment, the immunoglobulin Fc region is originated from humans.

In addition, the immunoglobulin Fc region may be an Fc region derived from IgG, IgA, IgD, IgE, IgM, or a combination or hybrid thereof. In a still more specific embodiment, the immunoglobulin Fc region is derived from IgG or IgM, which is most abundant in the human blood, and in a still more specific embodiment, the immunoglobulin Fc region is derived from IgG, which is known to increase the half-lives of ligand-binding proteins. In a still more specific embodiment, the immunoglobulin Fc region is an IgG4 Fc region, and in a most specific embodiment, the immunoglobulin Fc region is an aglycosylated Fc region derived from human IgG4, but is not limited thereto.

In a specific embodiment, the immunoglobulin Fc region, which is a human IgG4 Fc region, may be in the form of a homodimer in which two monomers are linked through a disulfide bond (an inter-chain form) between cysteines, which correspond to the amino acid at position 3 in each monomer. Particularly, each monomer of the homodimer independently has/or may have an internal disulfide bond between the cysteines at positions 35 and 95 and an internal disulfide bond between the cysteines at positions 141 and 199, that is, two internal disulfide bonds (intra-chain form). With respect to the number of amino acids, each monomer may consist of 221 amino acids, and the number of the amino acids forming the homodimer may be a total of 442, but the number of amino acids is not limited thereto. Specifically, the immunoglobulin Fc region may be in the form of a homodimer in which two monomers each having the amino acid sequence of SEQ ID NO: 70 (consisting of 221 amino acids) are linked to each other through a disulfide bond between cysteines, each of which is the amino acid at position 3 of each monomer, wherein the monomers of the homodimer have each independently an internal disulfide bond formed between cysteines at positions 35 and 95 and an internal disulfide bond formed between cysteines at positions 141 and 199, but is not limited thereto.

F in Chemical Formula 1 may include a monomer having the amino acid sequence of SEQ ID NO: 70, wherein F may be a homodimer of monomers each having the amino acid sequence of SEQ ID NO: 70, but is not limited thereto.

As one example, the immunoglobulin Fc region may be a homodimer including the amino acid sequence of SEQ ID NO: 71 (consisting of 442 amino acids), but is not limited thereto.

The term "combination" as used herein means that polypeptides encoding single-chain immunoglobulin Fc regions of the same origin are linked to a single-chain polypeptide of a different origin to form a dimer or multimer. That is, a dimer or multimer may be prepared from two or more fragments selected from the group consisting of IgG Fc, IgA Fc, IgM Fc, IgD Fc, and IgE Fc fragments.

The above-described conjugate may have an increased duration of efficacy compared with native glucagon, or X not modified with F, and such a conjugate includes not only the above-described forms but also a form that is encapsulated in a biodegradable nanoparticle.

A composition containing the liver-targeted drug, for example, a composition containing the peptide (e.g., the peptide itself or a long-acting conjugate in which an immunoglobulin Fc region is linked to the peptide), may be used for preventing or treating a disease requiring drug action in the liver.

The term "prevention" as used herein refers to any action that inhibits or delays the occurrence of a disease requiring drug action in the liver by administration of a composition containing the liver-targeted drug, and the term "treatment" as used herein refers to any action that alleviates or advantageously changes the symptoms of a disease requiring drug action in the liver by administration of a composition containing the liver-targeted drug.

The term "administration" as used herein refers to the introduction of a predetermined substance into a patient by any appropriate method, and the route of administration of the composition may include, but is not particularly limited to, any typical route, through which the composition can arrive at a target in vivo, and for example, the route of administration may be intraperitoneal administration, intravenous administration, intramuscular administration, subcutaneous administration, intradermal administration, oral administration, topical administration, intranasal administration, intrapulmonary administration, intrarectal administration, or the like.

The term "disease requiring drug action in the liver" refers to a disease, for which the liver-targeted drug according to the present invention can exhibit therapeutic activity, wherein a prevention or treatment effect of the drug can be exhibited by an increased distribution of the drug administered to the liver tissue. For example, the disease may be a liver disease, hypoglycemia, or congenital hyperinsulinism, but is not particularly limited thereto. Any type of disease that can be treated by targeting of the liver-targeted drug of the present invention is included in the disease of the present invention without limitation.

As disclosed in WO 2020/263063, peptides or long-acting conjugates thereof having activity to the glucagon receptor exhibit excellent effects on liver diseases, such as metabolic liver disease, simple steatosis, non-alcoholic fatty liver disease, liver inflammation, non-alcoholic steatohepatitis, cholestatic liver disease, liver fibrosis, liver cirrhosis, liver decompensation, and hepatocellular carcinoma. Therefore, the peptide or long-acting conjugate thereof having activity to the glucagon receptor, which is a liver-targeted drug of the present invention with excellent targeting to liver tissue, also has an effect of preventing or treating the liver diseases and can be effectively provided as a medicine for the liver diseases.

In the present invention, the term "liver disease" refers to a disease occurring in the liver, and the liver disease may be any one selected from the group consisting of nonalcoholic fatty liver disease (NAFLD), liver fibrosis, liver inflammation, liver cirrhosis, liver decompensation, hepatocellular carcinoma, and cholestasis liver disease. For example, the non-alcoholic fatty liver diseases may be a series of diseases including simple steatosis with only excessive accumulation of fat in liver cells, non-alcoholic fatty liver, non-alcoholic steatohepatitis (NASH) accompanied by hepatocyte necrosis, inflammation, and fibrosis, and the cholestatic liver disease may be primary biliary cirrhosis, primary sclerosing cholangitis, or a combination thereof, but the liver disease is not limited to the above-mentioned diseases as long as there are abnormalities in the tissue and functions of the liver.

The term "hypoglycemia" as used herein refers to a condition in which the blood glucose level is lower than those of normal people. The term typically refers to a condition in which the blood glucose level is 50 mg/dL or less, but is not particularly limited thereto. Hypoglycemia is frequently caused when a person taking an oral hypoglycemic agent or insulin consumes less than usual or engages in activities or exercises more than usual. Besides, hypoglycemia may be also caused by drinking or use of blood glucose level-lowering medications, severe physical illnesses, hormone deficiencies such as adrenocortical hormone and glucagon, insulin-producing pancreatic tumors, autoimmune diseases against insulin, patients with gastric resection, inborn errors of carbohydrate metabolism disorder, or the like.

In the present invention, the hypoglycemia encompasses both acute hypoglycemia and chronic hypoglycemia.

Symptoms of hypoglycemia include having no energy, body trembling, pallor, cold sweat, dizziness, excitement, anxiety, heart palpitations, feeling of hunger, headache, fatigue, and the like. If hypoglycemia persists for a long time, it may induce convulsion or seizure and cause shock, potentially resulting in fainting.

More specifically, the hypoglycemia may be caused by persistent hyperinsulinism due to a genetic defect. Examples of known causes of hyperinsulinism due to a genetic defect are known to include a mutation of SUR gene or Kir6.2 gene on chromosome 11p15.1, or an increase in glucokinase (GK) activity due to a mutation of GK gene on chromosome 7p15-p13, and an activation of GDH due to a mutation of glutamate dehydrogenase (GDH) gene and the resultant ATP increase in the islet cells.

Meanwhile, congenital hyperinsulinism is one of the causative diseases that results in severe and persistent hypoglycemia in newborns and children. The congenital hyperinsulinism may be caused by temporary increases in insulin secretion in low birth weight infants or infants of diabetic mothers, dysfunctions of pancreatic cells due to genetic mutations, and the like.

Meanwhile, it is known that the liver can produce glucose through glycogenolysis of stored glycogen and gluconeogenesis. Therefore, a more effective alleviation of hypoglycemia and normalization of blood sugar levels can be anticipated if a pharmaceutical composition capable of inducing glycogenolysis and gluconeogenesis in the liver, for example, glucagon and a conjugate including the same, is targeted to the liver in the event of hypoglycemia.

The pharmaceutical composition of the present invention may further contain a pharmaceutically acceptable carrier, excipient, or diluent. The pharmaceutically acceptable carrier, excipient, or diluent may be non-naturally occurring.

The term "pharmaceutically acceptable" as used herein refers to having a sufficient amount to exhibit a therapeutic effect and not causing adverse effects, and can be easily determined by a skilled person in the art based on the factors well known in the medical field, such as the type of disease, patient's age, body weight, health condition, and sex, drug sensitivity of a patient, route of administration, method of administration, frequency of administration, duration of treatment, drugs used in combination or concurrently.

The pharmaceutical composition containing the peptide of the present invention may further contain a pharmaceutically acceptable excipient. Although the carrier is not particularly limited to these, a binder, a lubricant, a disintegrant, a solubilizer, a dispersant, a stabilizer, a suspending agent, a coloring agent, a flavoring agent, and the like may be used for oral administration; a buffer, a preservative, an analgesic, a solubilizer, an isotonic agent, a stabilizer, and the like may be mixed for an injection; and a base, an excipient, a lubricant, a preservative, and the like may be used for topical administration.

The formulation of the composition of the present invention may be prepared in various manners by mixing with the above-described pharmaceutically acceptable carriers. For example, for oral administration, the composition of the present invention may be formulated in the form of a tablet, a troche, a capsule, an elixir, a suspension, a syrup, a wafer, or the like, and for the injection, the composition may be formulated as a single dose ampoule or a multidose form. The composition may be formulated into a solution, a suspension, a tablet, pills, a capsule, a sustained-release preparation, or the like.

Meanwhile, examples of the carrier, excipient, and diluent suitable for preparation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oils, and the like. In addition, the composition may further contain a filler, an anti-coagulant, a lubricant, a humectant, a flavoring agent, a preservative, and the like.

In addition, the pharmaceutical composition of the present invention may have any one formulation selected from the group consisting of a tablet, a pill, a powder, granules, a capsule, a suspension, a liquid medicine for internal use, an emulsion, a syrup, a sterile aqueous solution, a non-aqueous solvent, a lyophilized preparation, and a suppository.

The composition may be formulated into a single dose form suitable for the patient's body, and specifically, may be formulated into a preparation useful for the administration of a protein medicine, and may be administered by an administration method commonly used in the art through an oral route or a parenteral route including skin, intravenous, intramuscular, intra-arterial, intramedullary, intrathecal, intraventricular, pulmonary, transdermal, subcutaneous, intraperitoneal, intranasal, intragastric, topical, sublingual, vaginal, or rectal route, but the route of administration is not limited thereto.

In addition, the conjugate may be used by mixing with various carriers approved as medications, such as physiological saline or organic solvents, and for increasing stability or absorptivity, a carbohydrate such as glucose, sucrose, or dextran, an antioxidant such as ascorbic acid or glutathione, a chelating agent, a low-molecular weight protein, other stabilizers, or the like may be used as a medication.

The administration dose and frequency of the pharmaceutical composition of the present invention are determined according to the type of drug, which is an active ingredient, together with various factors, such as a disease to be treated, a route of administration, patient's age, sex, and body weight, and severity of a disease.

The total effective amount of the composition of the present invention may be administered to a patient in a single dose, or may be administered in multiple doses by a fractionated treatment protocol for a long period of time. In the pharmaceutical composition of the present invention, the content of the active ingredient may vary depending on the severity of a disease. Specifically, the total daily dose of the conjugate of the present invention may be about 0.0001 mg to 500 mg per kg of body weight of a patient. However, with respect to the dosage of the conjugate, the effective dose thereof is determined considering various factors, such as patient's age, body weight, health condition, and sex, the severity of a disease, a diet, and an excretion rate, in addition to the route of administration and the frequency of treatment of the pharmaceutical composition, and therefore, considering these, a person skilled in the art can easily determine an effective dose appropriate for particular use of the pharmaceutical composition of the present invention. The pharmaceutical composition according to the present invention is not particularly limited to the formulation, route of administration, and method of administration as long as the pharmaceutical composition exhibits the effects of the present invention.

The pharmaceutical composition of the present invention may have excellent *in vivo* duration and titer, thereby significantly reducing the number and frequency of administration of the pharmaceutical preparation of the present invention.

In accordance with another aspect of the present invention, there is provided a biologically active substance targeted to liver tissue, specifically, a biologically active substance targeted to liver tissue and containing a substance having binding affinity for the glucagon receptor.

The biologically active substance targeted to liver tissue, substance having binding affinity for the glucagon receptor, and the like are as described above.

In accordance with another aspect of the present invention, there is provided a method for preventing or treating a disease requiring drug action in the liver, the method including administering to a subject in need thereof the pharmaceutical composition or a biologically active substance targeted to liver tissue.

The pharmaceutical composition, biologically active substance targeted to liver tissue, disease requiring drug action in the liver, prevention, and treatment are as described above.

In the present invention, the subject refers to a subject suspected of having a disease requiring drug action in the liver, and the subject suspected of having a disease requiring drug action in the liver indicates mammals including humans, rats, livestock, and the like, which have or are at risk of developing the corresponding disease, but any subject that can be treated with the composition containing the liver-targeted drug of the present invention is included in the subject of the present invention without limitation.

The method of the present invention may include administering a pharmaceutically effective amount of the pharmaceutical composition containing the liver-targeted drug. The appropriate total daily dose may be determined by a practitioner within appropriate medical judgment, and may be administered once or several times in divided doses. For the purpose of the present invention, the specific therapeutically effective dose for a specific patient may be preferably applied differently depending on various factors well known in the medical art, including the type and degree of responses to be achieved, a specific composition including whether other agents are occasionally used therewith, the patient's age, body weight, general health condition, sex and a diet, the time of administration, the route of administration, the secretion rate of the composition, the duration of treatment, other drugs used in combination or concurrently with the composition of the present invention, and similar factors well-known in the medical art.

In accordance with another aspect of the present invention, there is provided use of the liver-targeted drug, specifically, a biologically active substance targeted to liver tissue, in the preparation of a medication for prevention or treatment of a disease requiring drug action in the liver.

The disease requiring drug action in the liver, liver-targeted drug, and biologically active substance targeted to liver tissue are as described above.

In accordance with another aspect of the present invention, there is provided a method for inducing targeting of a liver-targeted drug to the liver by administering a subject in need thereof the pharmaceutical composition, specifically, a biologically active substance targeted to liver tissue.

The liver-targeted drug, biologically active substance targeted to liver tissue, and subject are as described above.

In accordance with another aspect of the present invention, there is provided a method for inducing an increased distribution of a liver-targeted drug in liver tissue by administering a subject in need thereof the pharmaceutical composition, specifically, a biologically active substance targeted to liver tissue.

The liver-targeted drug, biologically active substance targeted to liver tissue, and subject are as described above.

In the method of inducing targeting to the liver, the corresponding substance can be administered through an appropriate route, for example, subcutaneous (s.c.) route, to be able to be targeted to the liver, but is not specifically limited thereto.

Hereinafter, the present invention will be described in detail with reference to the following examples. However, the following examples are merely for illustrating the present invention and are not intended to limit the scope of the present invention.

### Example 1: Preparation of glucagon and its derivative peptides

Glucagon and its derivative peptides that exhibit activity to the glucagon receptor were prepared, and the sequences thereof are shown in Table 1 below. Specifically, the amino acid sequence of native glucagon of SEQ ID NO: 1 was subjected to substitution with negatively and positively charged amino acid residues to synthesize glucagon derivative peptides shown in Table 1 below. Then, the relative *in vitro* activities thereof were measured by the method described in Experimental Example 1 below.

**TABLE 1**

| SEQ ID NO | Sequences of glucagon and its derivative | Ring formation or non-formation | pl | *In vitro* activity (relative activity compared with SEQ ID NO: 1, %) |
|---|---|---|---|---|
| SEQ ID NO: 1 | HSQGTFTSDYSKYLDSRR AQDFVQWLMNT | - | 6.8 | 100 |
| SEQ ID NO: 2 | HSQGTFTSDYSKYLDCDR AQDFVQWLMNT | - | 4.56 | 0.6 |
| SEQ ID NO: 3 | HSQGTFTSDYSKYLDCER AQDFVQWLMNT | - | 4.66 | 6.1 |
| SEQ ID NO: 4 | HSQGTFTSDYSKYLDSCD AQDFVQWLMNT | - | 4.13 | < 0.1 |
| SEQ ID NO: 5 | HSQGTFTSDYSKYLDSCE AQDFVQWLMNT | - | 4.22 | 0.3 |
| SEQ ID NO: 6 | HSQGTFTSDYSKYLDSCE ADDFVQWLMNT | - | 4.03 | < 0.1 |
| SEQ ID NO: 7 | YSQGTFTSDYSKYLDSCE ADDFVQWLMNT | - | 3.71 | < 0.1 |
| SEQ ID NO: 8 | YXQGTFTSDYSKYLDSCD AQDFVQWLINT | - | 3.77 | < 0.1 |
| SEQ ID NO: 9 | YXQGTFTSDYSKYLDSCD AQDFVVWLINT | - | 3.77 | < 0.1 |
| SEQ ID NO: 10 | YXQGTFTSDYSKYLDSCD ADDFVVWLINT | - | 3.66 | < 0.1 |
| SEQ ID NO: 11 | YXQGTFTSDYSKYLDEKC AKEFVQWLMNT | - | 4.78 | 4.6 |
| SEQ ID NO: 12 | YXQGTFTSDYSKYLDEKR AKEFVQWLMNTC | Ring formation | 6.20 | 56.3 |
| SEQ ID NO: 13 | YXQGTFTSDYSCYLDSRR AQDFVQWLMNT | - | 4.43 | 5.2 |
| SEQ ID NO: 14 | YXQGTFTSDYSKYLDCKR AKEFVQWLMNT | - | 8.12 | 18.1 |
| SEQ ID NO: 15 | YXQGTFTSDYSKYLCEKR AQDFVVWLMNT | - | 6.11 | 1.1 |
| SEQ ID NO: 16 | YXQGTFTSDYSKYLDCRR AQVFVQWLMRT | - | 9.11 | 4.2 |
| SEQ ID NO: 17 | YXQGTFTSDYSKYLDCVR AQDFVQWLMRT | - | 6.03 | 23.2 |
| SEQ ID NO: 18 | YXQGTFTSDYSKYLDSRR | - | 8.15 | < 0.1 |
| | ACDFRLWVLMNT | | | |
| SEQ ID NO: 19 | YXQGTFTSDYSKYLCEKR AKEFVQWLMNT | Ring formation | 8.12 | 12.1 |
| SEQ ID NO: 20 | YXQGTFTSDYSKYLDECR AKEFVQWLMNT | Ring formation | 4.78 | 299.7 |
| SEQ ID NO: 21 | YXQGTFTSDYSKYLDEKC AKEFVQWLMNT | Ring formation | 4.78 | 57.8 |
| SEQ ID NO: 22 | YXQGTFTSDYSKYLDEKR CKEFVQWLMNT | Ring formation | 6.20 | 147.8 |
| SEQ ID NO: 23 | YXQGTFTSDYSKYCDEKR AKEFVQWLMNT | Ring formation | 6.20 | 76.8 |
| SEQ ID NO: 24 | YXQGTFTSDYSKCLDEKR AKEFVQWLMNT | Ring formation | 6.21 | 58.0 |
| SEQ ID NO: 25 | YXQGTFTSDYSKYLDEKR AKCFVQWLMNT | Ring formation | 8.12 | 46.9 |
| SEQ ID NO: 26 | WXQGTFTSDYSKYLDECR AKDFVQWLMNT | Ring formation | 4.68 | 1.0 |
| SEQ ID NO: 27 | YXQGTFVSDYSKYLDECR AKDFVQWLMNT | Ring formation | 4.68 | 93.6 |
| SEQ ID NO: 28 | WXQGTFVSDYSKYLDECR AKDFVQWLMNT | Ring formation | 4.68 | < 0.1 |
| SEQ ID NO: 29 | YXQGTFTSDYSKCLDERR AKDFVQWLMNT | Ring formation | 6.15 | 61.3 |
| SEQ ID NO: 30 | WXQGTFTSDYSKCLDERR AKDFVQWLMNT | Ring formation | 4.44 | 0.3 |
| SEQ ID NO: 31 | YXQGTFTSDYSKYLDCKR AKEFVQWLMNT | Ring formation | 8.12 | 6.3 |
| SEQ ID NO: 32 | - | Ring formation | 4.78 | 0.7 |
| | SQGTFTSDYSKYLDECRA KEFVQWLMNT | | | |
| SEQ ID NO: 33 | YXQGTFTSDYSKYLDSRR AQDFVQWLMNT | - | 6.04 | 108.2 |
| SEQ ID NO: 34 | WXQGTFTSDYSKYCDER RAKEFVQWLMNT | Ring formation | 6.21 | 0.2 |
| SEQ ID NO: 35 | YXQGTFTSDYSKYCDERR AKEFVQWLMNT | Ring formation | 6.2 | 17.7 |
| SEQ ID NO: 36 | YXQGTFTSDCSKYLDERR AKEFVQWLMNT | Ring formation | 6.21 | 9.9 |
| SEQ ID NO: 37 | YXQGTFTSDYSKYLDERR AKEFVQWLMNTC | Ring formation | 6.21 | 225.5 |
| SEQ ID NO: 38 | YXQGTFCSDYSKYLDERR AKEFVQWLMNT | Ring formation | 6.15 | 167.3 |
| SEQ ID NO: 39 | YXQGTFVSDCSKYLDERR AKDFVQWLMNT | Ring formation | 6.15 | 3.7 |
| SEQ ID NO: 40 | YXQGTFVSDYSKYLDERR AKDFVQWLMNTC | Ring formation | 6.15 | 40.8 |
| SEQ ID NO: 41 | YXQGTFCSDYSKYLDERR AKDFVQWLMNT | Ring formation | 6.03 | 45.2 |
| SEQ ID NO: 42 | YXQGTFCSDYSKYLDSRR AQDFVQWLMNT | - | 6.03 | 37.9 |
| SEQ ID NO: 43 | YXQGTFTSDCSKYLDSRR AQDFVQWLMNT | - | 6.03 | 1.6 |
| SEQ ID NO: 44 | YXQGTFTSDYSKYLDSRR AQDFVQWLMNTC | - | 6.21 | 75.4 |
| SEQ ID NO: 45 | YXQGTFTSDYSCYLDEKR AKEFVQWLMNT | - | 4.78 | 5.2 |

In the amino acids sequences shown in Table 1, the amino acid marked by X represents the non-native amino acid aminoisobutyric acid (Aib), the underlines under amino acid symbols represent the formation of a lactam ring between the side chains of a pair of the corresponding amino acids, and the symbol "-" represents the absence of an amino acid residue at a corresponding position. Additionally, on the column with respect to ring formation or non-formation, the symbol "-" indicates that there is ring non-formation in the corresponding sequence.

### Example 2: Production of long-acting conjugates of glucagon derivative peptides

As a representative glucagon derivative peptide, the glucagon derivative of SEQ ID NO: 37 was selected among the derivative peptides in Table 1 prepared in Example 1, and a conjugate thereof was prepared as follows.

Maleimide-PEG-aldehyde (NOF, Japan), a linearly modified polyethylene glycol with a molecular weight of 10 kDa, in which the hydrogens at both ends are substituted with a 3-(3-maleimidopropionamido)propyl group and a 3-oxopropyl group (propionaldehyde group), respectively, was reacted with the glucagon derivative peptide of SEQ ID NO: 37 retaining cysteine to PEGylate the cysteine residue of the glucagon derivative peptide at the maleimide end of the maleimide-PEG-aldehyde.

Specifically, the glucagon derivative peptide of SEQ ID NO: 37 and maleimide-PEG-aldehyde were reacted at a molar ratio of 1:1-5, with a protein concentration of 3-10 mg/mL, at a low temperature for 1-3 hours. Particularly, the reaction was conducted in an environment where 20-60% isopropanol was added to 50 mM Tris buffer (pH 7.5). Upon completion of the reaction, the reaction solution was applied to SP sepharose HP (GE Healthcare, USA) to purify the glucagon derivative mono-PEGylated on cysteine.

An immunoglobulin Fc fragment was prepared using the immunoglobulin Fc fragment (49.8 kDa, a homodimer in which two chains of SEQ ID NO: 70 are linked by a disulfide bond) having a hinge region of the Pro-Ser-Cys-Pro sequence (SEQ ID NO: 60) at the N-terminus by the method disclosed in International Patent Publication No. WO 2007/021129.

Then, the purified mono-PEGylated glucagon derivative peptide and the immunoglobulin Fc fragment were reacted at a molar ratio of 1:2-10, with a protein concentration of 10-50 mg/mL, at 4-8°C for 12-18 hours. The reaction was conducted in an environment where 10-50 mM sodium cyanoborohydride and 10-20% isopropanol as reductants were added to 100 mM potassium phosphate buffer (pH 6.0). Upon completion of the reaction, the reaction solution was applied to the Butyl Sepharose FF purification column (GE Healthcare, USA) and Source ISO purification column (GE Healthcare, USA) to purify a long-acting conjugate of the glucagon derivative peptide, in which the polyethylene glycol end at the aldehyde side of the mono-PEGylated glucagon derivative peptide is linked to nitrogen at the N-terminal proline of one of the two chains of the immunoglobulin Fc homodimer.

After the preparation, the purity of the conjugate was 95% or more as analyzed by reverse-phase chromatography, size exclusion chromatography, and ion exchange chromatography.

Particularly, the conjugate where the glucagon derivative peptide and the immunoglobulin Fc fragment are linked via PEG was designated as "long-acting conjugate of glucagon derivative peptide", "conjugate including glucagon derivative and immunoglobulin Fc", "long-acting conjugate of glucagon derivative", or "long-acting glucagon derivative", which can be used interchangeably with each other herein.

### Experimental Example 1: Measurement of in vitro activities of long-acting conjugates of glucagon derivatives

To determine activities of the glucagon derivatives prepared in Example 1, a method of measuring *in vitro* cell activity was employed for cell lines, into which the glucagon (GCG) receptor was transformed.

Each of the cell lines was transformed to express the human GCG receptor in Chinese hamster ovary (CHO) and suitable for measuring the activity of GCG. Therefore, the activity to the GCG receptor was measured using each of the transformant cell lines.

PCR was performed using, as a template, a portion corresponding to ORF in the cDNA (OriGene Technologies, Inc., USA) of the human glucagon receptor gene, along with the following forward and reverse primers of SEQ ID NOS: 48 and 49 including EcoRI and Xhol cleavage sites, respectively.

Particularly, PCR was performed by 30 cycles of denaturation at 95°C for 60 seconds, annealing at 55°C for 60 seconds, and extension at 68°C for 30 seconds. The amplified PCR products were electrophoresed on a 1.0% agarose gel, and then a 450-bp band was obtained by elution.
Forward primer (SEQ ID NO: 48)
   5'-CAGCGACACCGACCGTCCCCCCGTACTTAAGGCC-3'
Reverse primer (SEQ ID NO: 49)
   5'-CTAACCGACTCTCGGGGAAGACTGAGCTCGCC-3'

The PCR product was cloned into x0GC/dhfr, a known animal cell expression vector, to prepare the recombinant vector x0GC/GCGR.

The prepared recombinant vector x0GC/GCGR was transformed into CHO DG44 cells cultured in DMEM/F12 medium containing 10% FBS by using Lipofectamine, and selectively cultured in a selection medium containing 1 mg/mL G418 and 10 nM methotrexate. Single clone cell lines were selected therefrom by a limit dilution technique, and a cell line showing excellent cAMP response to glucagon in a concentration-dependent manner was finally selected therefrom.

The activities of the glucagon derivatives synthesized in Example 1 were measured in the cell line. Specifically, the transformant cell line was subcultured three or four times one week, dispensed in a 384-well plate at 6×10³ cells per well, and then cultured for 24 hours. With respect to the cultured cells, the native glucagon at 200 nM and the glucagon derivatives at 1600 nM were suspended individually in Hank's balanced salt solution (HBSS) buffer containing 0.5 mM 3-isobutyl-1-methylxanthine (IBMX), 0.1% bovine serum albumin (BSA), and 5 mM 4-(2-hydroxyethyl)-1-piperazineethanesulfonic acid (HEPES), serially diluted 4-fold ten times, applied to a cAMP assay kit (LANCE cAMP 384 kit, PerkinElmer), and added to the cultured cells, and then the fluorescence value was measured. After the measurement, the highest fluorescence value was set to 100%, based on which the EC₅₀ values of the glucagon derivatives were calculated, followed by comparison with that of native glucagon. The results are shown in Table 1 above.

The prepared glucagon derivatives have high activity to the glucagon receptor, and thus exhibit increased targeting to liver cells and can furthermore be used as therapeutic substances for desired diseases in the liver by activating the glucagon receptor.

### Experimental Example 2: Measurement of pl of glucagon derivatives

In order to investigate the improved properties of the glucagon derivatives synthesized in Example 1, the pl values were estimated from the amino acid sequences by using the pl/Mw tool (http://expasy.org/tools/pi_tool.html; Gasteiger et al., 2003) in the ExPASy server.

As shown in Table 1 above, the native glucagon of SEQ ID NO: 1 had a pl of 6.8, while some glucagon derivatives according to the present invention showed pl values ranging from about 4 to about 6. These glucagon derivatives have higher or lower pl values than native glucagon and thus can exhibit improved solubility and higher stability at a neutral pH condition compared with native glucagon.

The glucagon derivatives according to the present invention, when used as therapeutic agents for targeted diseases in the liver, can exhibit increased patient compliance.

### Experimental Example 3: Investigation of tissue distribution of long-acting conjugate of glucagon derivative

In three SD rats, the distributions of the long-acting conjugate of SEQ ID NO: 37, selected as a representative example among the long-acting conjugates of glucagon derivatives, in tissues and organs were compared.

Specifically, the long-acting conjugate of the glucagon derivative was subcutaneously injected at 1558 µg/kg for each case, and then the organs were extracted at 4, 48, and 168 hours, and thereafter, the substance concentrations in respective tissues (serum, brain, pancreas, heart, kidney, stomach, small intestine, large intestine, lung, liver, spleen, adipose tissue, and muscle) were measured and compared by an ELISA method. The T/S ratio (%) was calculated as tissue concentration/serum concentration*100, and for example, the 48-hour data in Table 3 below were calculated as 15500.8 ± 2686.9 in serum, 4625.4 ± 1216.9 in liver, and thus the T/S ratio (%) was calculated as 4625.4/15500.8*100, which was about 29.8%. For concentration measurement, each organ was extracted, and then the concentration of the substance was measured using ELISA.

As a result, the tissue distributions of the long-acting conjugate of the glucagon derivative were most strongly detected 48 hours after administration, and especially, showed a high distribution ratio in the liver. The tissue distribution was higher in that order of the liver, heart, lung, large intestine, spleen, small intestine, muscle, stomach, pancreas, adipose tissue, and kidney. The substance was still detected at the highest ratio in the liver even 168 hours after administration, indicating that the substance remained at a high fraction in the liver even 7 days after administration. In addition, the substance was present at the highest ratio in the liver 168 hours after administration, and the distribution ratio of the substance was higher in the lung than the heart when compared with those at 48 hours after administration. Table 2 below summarizes the results of tissues to serum distribution ratios of the long-acting conjugate of SEQ ID NO: 37 as confirmed in the above example.

**TABLE 2**

| Tissue | 4 hours | | 48 hours | | 168 hours | |
|---|---|---|---|---|---|---|
| | Conc (ng/ml, ng/g) | T/S (%) | Conc (ng/ml, ng/g) | T/S (%) | Conc (ng/ml, ng/g) | T/S (%) |
| Serum | 416.0 ± 284.2 | - | 15500.8 ± 2686.9 | - | 6534.2 ± 2070.3 | - |
| Liver | NA | NA | 4625.4 ± 1216.9 | 29.8 | 2027.6 ± 444.5 | 31 |
| Heart | NA | NA | 2447.4 ± 261.9 | 15.8 | 615.9 ± 124.3 | 9.4 |
| Lung | NA | NA | 1820.5 ± 258.7 | 11.7 | 703.1 ± 175.5 | 10.8 |
| Large intestine | NA | NA | 1411.9 ± 222.9 | 9.1 | 221.1 ± 11.0 | 3.4 |
| Spleen | NA | NA | 1323.7 ± 582.3 | 8.5 | 518.2 ± 29.5 | 7.9 |
| Pancreas | NA | NA | 499.8 ± 331.7 | 3.2 | NA | NA |
| Adipose tissue | NA | NA | 453.9 ± 107.9 | 2.9 | NA | NA |
| Small intestine | NA | NA | 785.5 ±137.9 | 5.1 | NA | NA |
| Stomach | NA | NA | 598.1 ± 152.9 | 3.9 | NA | NA |
| Muscle | NA | NA | 735.4±193.3 | 4.7 | 220.2±30.3 | 3.4 |
| Kidney | NA | NA | 284.9 ± 105.5 | 1.8 | NA | NA |
| Brain | NA | NA | NA | NA | NA | NA |

| | | | | | | |
|---|---|---|---|---|---|---|
| * T/S = tissue-to-serum ratio, * NA = Not applicable * ng/ml indicates concentration in serum, and ng/g indicates concentration in tissue | | | | | | |

The above results suggest that the long-acting conjugate of a glucagon derivative of the present invention showed excellent tissue distribution in the liver compared with the other tissues, indicating that the long-acting conjugates of glucagon derivatives can be used as a therapeutic substance for a target disease. Therefore, long-acting conjugates of glucagon derivatives can be used for novel use for optimization of drug treatment by inducing targeting to liver tissue and allowing a necessary amount of drug to be effectively delivered thereto.

Furthermore, the above results suggest that the distribution in the liver remained high even 7 days after administration, supporting the convenience of the drug as a preparation that can be administered about once a week.

While the present invention has been described with reference to the particular illustrative embodiments, a person skilled in the art to which the present invention pertains can understand that the present invention may be embodied in other specific forms without departing from the technical spirit or essential characteristics thereof. In this regard, the embodiments described above should be understood to be illustrative rather than restrictive in every respect. The scope of the invention should be construed to mean that the meaning and scope of the appended claims rather than the detailed description and all changes or variations derived from the equivalent concepts fall within the scope of the present invention.

### [DESCRIPTION OF GOVERNMENT-SPONSORED RESEARCH]

This study was supported by the National New Drug Development Project Support of the Korea Drug Development Fund funded by the Ministry of Science and ICT, the Ministry of Trade, Industry and Energy, and the Ministry of Health and Welfare (HN21C0601).

## Claims

1. A pharmaceutical composition comprising a liver-targeted drug and having a high distribution of the drug in the liver among the organs in the body of a subject administered the drug, wherein the liver-targeted drug is a peptide including an amino acid sequence of General Formula 2 below:
Y-Aib-QGTF-X7-SD-X10-S-X12-Y-L-X15-X16-X17-R-A-X20-X21-F-V-X24-W-L-M-N-T-X30 (General Formula 2, SEQ ID NO: 47),
wherein in the General Formula 2 above,
X7 is threonine (T), valine (V), or cysteine (C);
X10 is tyrosine (Y) or cysteine (C);
X12 is lysine (K) or cysteine (C);
X15 is aspartic acid (D) or cysteine (C);
X16 is glutamic acid (E) or serine (S);
X17 is lysine (K) or arginine (R);
X20 is glutamine (Q) or lysine (K);
X21 is aspartic acid (D) or glutamic acid (E);
X24 is valine (V) or glutamine (Q); and
X30 is cysteine (C) or is absent
(with the proviso that an amino acid sequence of General Formula 2 identical to SEQ ID NO: 12 is excluded).

2. The pharmaceutical composition of claim 1, wherein the peptide is in the form of a long-acting conjugate, and the long-acting conjugate is represented by chemical formula 1 below:
[Chemical Formula 1] X-L-F
wherein,
X represents a peptide including an amino acid sequence of General Formula 2;
L represents a linker containing ethylene glycol repeating units;
F represents an immunoglobulin Fc region; and
the "-" symbols represent covalent linkages between X and L and between L and F, respectively.

3. The composition of claim 1 or 2, wherein the organs in the body are liver, heart, lung, large intestine, spleen, pancreas, adipose tissue, small intestine, stomach, muscle, kidney, and brain, among which the distribution of the drug is highest in the liver.

4. The pharmaceutical composition of claim 1 or 2, wherein the pharmaceutical composition is used for the prevention or treatment of a disease requiring drug action in the liver.

5. The pharmaceutical composition of claim 1 or 2, wherein the tissue-to-serum ratio (T/S ratio) of the liver-targeted drug in the liver after administration is at least one selected from below:
(a) a T/S ratio of 20-40% at 40-50 hours after administration; and
(b) a T/S ratio of 25-40% at 160-180 hours after administration.

6. The pharmaceutical composition of claim 5, wherein the T/S ratio of the liver-targeted drug in the liver after administration is at least one selected from below:
(a) a T/S ratio of 25-35% at 2 days after administration; and
(b) a T/S ratio of 27-37% at 7 days after administration.

7. The pharmaceutical composition of claim 1 or 2, wherein the liver-targeted drug shows a distribution ratio of 1:2-4 in the liver to lung tissue after administration.

8. The pharmaceutical composition of claim 7, wherein the liver-targeted drug shows a distribution ratio of 1:2.2-3.2 in the liver to lung tissue after administration.

9. The pharmaceutical composition of claim 7, wherein the distribution ratio is a distribution ratio at 40-180 hours after administration.

10. The pharmaceutical composition of claim 8,
wherein the distribution ratio is a distribution ratio at 2 days to 7 days after administration.

11. The pharmaceutical composition of claim 1 or 2, wherein the liver-targeted drug shows:
(a) a distribution ratio of 1:1.6-3.0 in the liver to heart at 40-50 hours after administration; and
(b) a distribution ratio of 1:2.8-7.0 in the liver to heart at 160-180 hours after administration;

12. The pharmaceutical composition of claim 1 or 2, wherein the liver-targeted drug has therapeutic activity on a liver disease, hypoglycemia, or congenital hyperinsulinism.

13. The pharmaceutical composition of claim 4, wherein the disease requiring drug action in the liver is a liver disease, hypoglycemia, or congenital hyperinsulinism.

14. The pharmaceutical composition of claim 13, wherein the hypoglycemia is acute hypoglycemia or chronic hypoglycemia.

15. The pharmaceutical composition of claim 1 or 2, wherein a ring is formed between the amino acids of X16 and X20 in General Formula 2.

16. The pharmaceutical composition of claim 1 or 2, wherein the peptide include an amino acid sequence selected from the group consisting of SEQ ID SEQ ID NOS: 13, 15, 19, 33, and 36-45.

17. The pharmaceutical composition of claim 1 or 2, wherein the peptide include an amino acid sequence selected from the group consisting of SEQ ID NOS: 33 and 36-44.

18. The pharmaceutical composition of claim 1 or 2, wherein the C-terminus of the peptide is amidated or has a free carboxyl group (-COOH).

19. The pharmaceutical composition of claim 1 or 2, wherein the C-terminus of the peptide is amidated.,

20. The pharmaceutical composition of claim 1 or 2, wherein the C-terminus of the peptide is not modified.

21. The pharmaceutical composition of claim 2, wherein L is polyethylene glycol.

22. The pharmaceutical composition of claim 2, wherein the chemical formula weight of ethylene glycol repeating units in L is in the range of 1 to 100 kDa.

23. The pharmaceutical composition of claim 2, wherein F is an IgG Fc region.

24. A method for inducing targeting of a liver-targeted drug to the liver by administering a subject in need thereof the pharmaceutical composition of any one of claims 1 to 23.

25. A method for inducing an increased distribution of a liver-targeted drug in liver tissue by administering a subject in need thereof the pharmaceutical composition of any one of claims 1 to 23.
